# EUROPEAN PATENT SPECIFICATION

(11) **EP 4 133 964 B1**
(45) Date of publication and mention of the grant of the patent: **25.03.2026**
(21) Application number: 20929828.0
(22) Date of filing: 11.09.2020
(51) Int. Cl.: A45D 20/12, A61H 39/04, A61N 5/06

(54) **DIFFUSER AND HAIRDRYER INCLUDING SAME**
DIFFUSOR UND HAARTROCKNER DAMIT
DIFFUSEUR ET SÈCHE-CHEVEUX COMPRENANT CELUI-CI

(30) Priority: 10.04.2020 KR 20200044034
(43) Date of publication of application: 15.02.2023
(73) Proprietor: LG Electronics, Inc., Yeongdeungpo-gu Seoul 07336 (KR)
(72) Inventor: KIM, Kyoungtae, Seoul 08592 (KR); KIM, Hyunchul, Seoul 08592 (KR); KU, Yunhee, Seoul 08592 (KR); PARK, Rayoung, Seoul 08592 (KR); KIM, Dongwon, Seoul 08592 (KR)
(74) Representative: Vossius & Partner Patentanwälte Rechtsanwälte mbB
(86) International application number: PCT/KR2020/012282
(87) International publication number: WO 2021/206230

(56) References cited:
- CN-A- 109 480 440
- GB-A- 2 539 440
- JP-A- 2012 019 865
- JP-U- S6 455 803
- JP-U- S6 455 803
- KR-A- 20010 086 200
- KR-A- 20180 017 193
- KR-A- 20200 033 115
- KR-A- 20200 033 115
- KR-U- 19980 026 628
- KR-U- 20180 003 263

## Description

### [Technical Field]

The present disclosure relates to a diffuser and a hair dryer including the same, more particularly, to a diffuser coupled to a main body of a hair dryer to provide gas to a user and the hair dryer including the same.

### [Background Art]

When removing moisture from wet hair of a human body as much as desired or when styling the hair from a current shape to a desired shape, a hair dryer that discharges gas through a gas outlet portion may be used.

In one example, the hair dryer may provide gas characteristics desired by a user, such as a gas temperature, a gas speed, a gas flow area, and the like through a diffuser. The diffuser may be coupled to a main body of the hair dryer to change the gas characteristics and provide the changed gas characteristics to the user. Further, the diffuser may include care means such as a massage protrusion to manage a scalp health and the like of the user.

Korean Utility Model Application Publication No. 20-2011-0002484 discloses a diffuser provided in a hair dryer. The diffuser may be different from a gas outlet in a flow cross-sectional area of discharge gas or a speed of the discharge gas.

Therefore, it is an important task for the diffuser disposed on a main body of the hair dryer to efficiently provide the gas while providing a sufficient scalp or hair care effect to the user.

GB 2 539 440 A discloses a diffuser comprising a baffle which has an air inlet end, an air outlet end and an outwardly tapering wall extending between the inlet and outlet ends and together with a grille defining an air chamber.

### [Disclosure]

### [Technical Problem]

Embodiments of the present invention are to provide a diffuser and a hair dryer including the same capable of efficiently improving gas flow and providing the improved gas flow to a user.

In addition, embodiments of the present invention are to provide a diffuser and a hair dryer including the same in which structural stability may be effectively improved and manufacturing and assembly processes may be efficiently improved.

### [Technical Solution]

The present invention is defined by the appended independent claims, and preferred aspects of the present invention are defined by the appended dependent claims. A diffuser according to an embodiment of the present invention is coupled to a main body of a hair dryer to disperse gas provided from the main body and discharge the gas to outside.

In an embodiment of the present invention, flow dispersion of the gas may be achieved through a guide frame. The guide frame may be disposed inside the diffuser and guide the flow of the gas provided from the main body to be dispersed.

In an embodiment of the present invention, an entirety of a gas outlet disposed on the main body may be in a shape of a hole, or the gas may be respectively discharged from a center portion and a side portion spaced apart from the center portion.

The diffuser according to an embodiment of the present invention includes the guide frame therein, and the guide frame effectively diffuses the gas and guide the flow of the gas even when the gas is discharged from the entirety of the gas outlet or the separate gas flows are respectively provided from the center portion and the side portion.

The guide frame is disposed to diffuse the gas discharged from the gas outlet and may define a first flow path and a second flow path using a first guide and a second guide.

The gas provided from the gas outlet flows through the first flow path and the second flow path and is discharged forwardly of the diffuser. The gas discharged from the gas outlet may be diffused in the process of flowing into the first flow path and the second flow path, so that gas with improved uniformity may be discharged forwardly of the diffuser in a larger flow area.

The diffuser as described above includes a diffusing case and the guide frame. The diffusing case has a rear side removably coupled to the main body of the hair dryer, gas discharged from the main body is introduced into the diffusing case through a gas inlet hole defined at the rear side, the gas introduced into the diffusing case is discharged from a front side of the diffusing case, and an inner diameter of the diffusing case increases forwardly.

The guide frame is disposed inside the diffusing case to guide flow of the gas introduced through the gas inlet hole, The guide frame includes a diffusion portion, and the diffusion portion is disposed to face the gas inlet hole to diffuse the gas introduced through the gas inlet hole.

The gas flows inside the diffusing case where the inner diameter increases forwardly and diffuses in a radial direction of the diffusing case, so that the gas may be provided to a user with a wider flow cross-sectional area.

In addition, the guide frame includes the diffusion portion, and the diffusion portion is disposed to diffuse the gas introduced through the gas inlet hole in the radial direction, so that the gas introduced into the diffusing case is effectively diffused in the radial direction, thereby providing the gas with the uniform and wide flow cross-sectional area to the user.

The diffusion portion has a diffusion protrusion protruding toward the gas inlet hole on a rear surface of the diffusion portion facing the gas inlet hole, and a protrusion height of the diffusion protrusion may increase centerwardly.

The guide frame has a ring shape and includes a plurality of gas flow paths having the diffusion portion located at a center of the plurality of gas flow paths, and the plurality of gas flow paths are arranged to have different diameters to divide gas flows respectively in the plurality of gas flow paths from each other.

At least one of the plurality of gas flow paths may have a diameter larger than a diameter of the gas inlet hole.

The plurality of gas flow paths may include a first flow path and a second flow path. The guide frame may further include a first guide formed in a ring shape to have the diffusion portion located at a center of the first guide, wherein the first flow path is defined between the first guide and the diffusion portion, and a second guide formed in a ring shape to have the diffusion portion and the first guide at a center of the second guide, wherein the second flow path is defined between the second guide and the diffusion portion. The gas introduced through the gas inlet hole may flow to the front side of the diffusing case through the first flow path and the second flow path.

The second flow path may have an outer diameter larger than a diameter of the gas inlet hole, so that the gas introduced through the gas inlet hole may diffuse and flow.

The first guide may be located forwardly of the diffusion portion, and the second guide may be located forwardly of the first guide.

The diffuser may further include a light irradiator disposed on front surfaces of the diffusion portion, the first guide, and the second guide to irradiate light toward the front side of the diffusing case.

The guide frame may further include a guide connector extending along a radial direction of the guide frame to connect the diffusion portion, the first guide, and the second guide to each other.

The diffuser may further include a discharge cover disposed at the front side of the diffusing case, wherein the discharge cover includes a gas discharge hole for discharging the gas introduced into the diffusing case to outside, wherein the discharge cover may include a massage protrusion protruding forward.

In one example, a hair dryer according to an embodiment of the present invention includes a main body, a handle, and a diffuser. The main body includes a gas outlet for discharging gas therethrough, the handle extends from the main body, and a diffuser is removably coupled to the main body to introduce the gas discharged from the gas outlet therein and discharge the gas introduced therein to outside.

The diffuser includes a diffusing case having a rear side removably coupled to the main body, wherein the gas discharged from the gas outlet is introduced into the diffusing case through a gas inlet hole defined at the rear side, wherein the gas introduced into the diffusing case is discharged from a front side of the diffusing case, and wherein an inner diameter of the diffusing case increases forwardly, and a guide frame disposed inside the diffusing case to guide flow of the gas introduced through the gas inlet hole.

The guide frame includes a diffusion portion disposed to face the gas inlet hole to diffuse the gas introduced through the gas inlet hole.

The gas outlet may include a center portion for discharging the gas therethrough, and a side portion having a ring shape and surrounding the center portion, wherein the gas is discharged through the side portion along with the center portion. The diffusion portion may be disposed to face the center portion and have a diameter larger than a diameter of the center portion to diffuse the gas introduced from the center portion.

A diameter of the gas inlet hole may be equal to or larger than a diameter of the side portion, so that the gas discharged from the center portion and the side portion may flow into the diffusing case.

The guide frame may further include a first guide formed in a ring shape to have the diffusion portion located at a center of the first guide, wherein a first flow path for flowing the gas therethrough is defined between the first guide and the diffusion portion, and a second guide formed in a ring shape to have the diffusion portion and the first guide at a center of the second guide, wherein a second flow path for flowing the gas therethrough is defined between the second guide and the diffusion portion.

The diffusion portion may have a diameter smaller than an inner diameter of the side portion, and the side portion may be positioned to face at least a portion of the first flow path in a forward direction.

The second flow path may have an inner diameter larger than an outer diameter of the side portion, so that the gas introduced through the center portion and the side portion may be diffused and flow.

### [Advantageous Effects]

Embodiments of the present invention may provide the diffuser and the hair dryer including the same capable of efficiently improving the gas flow and providing the improved gas flow to the user.

In addition, embodiments of the present invention may provide the diffuser and the hair dryer including the same in which the structural stability may be effectively improved and the manufacturing and assembly processes may be efficiently improved.

### [Description of Drawings]

FIG. 1 is a view showing a hair dryer according to an embodiment of the present invention;
FIG. 2 is a view showing a state in which a diffuser is separated from a hair dryer shown in FIG. 1;
FIG. 3 is a view showing an internal cross-section of a hair dryer shown in FIG. 2;
FIG. 4 is a view showing a gas outlet in a hair dryer according to an embodiment of the present invention;
FIG. 5 is a view showing a diffuser according to an embodiment of the present invention;
FIG. 6 is a view showing an exploded view of a diffuser according to an embodiment of the present invention;
FIG. 7 is a view showing an internal cross-section of a diffuser according to an embodiment of the present invention;
FIG. 8 is a view showing a guide frame of a diffuser according to an embodiment of the present invention;
FIG. 9 is a view showing a guide frame shown in FIG. 8 when viewed from a side; and
FIG. 10 is a view showing an interior of a diffuser coupled to a main body according to an embodiment of the present invention.

### [Best Mode]

Hereinafter, an embodiment of the present invention will be described in detail with reference to the accompanying drawings to be easily implemented by those skilled in the art to which the present invention belongs.

However, the present invention may be implemented in many different forms and is not limited to embodiments described herein. In addition, in order to clearly describe the present invention, components irrelevant to the description are omitted, and like reference numerals are assigned to similar components throughout the specification.

In this specification, duplicate descriptions of the same components are omitted.

Further, in this specification, it will be understood that when a component is referred to as being "connected with" another component, the component may be directly connected with the other component or intervening components may also be present. In contrast, it will be understood that when a component is referred to as being "directly connected with" another component in this specification, there are no intervening components present.

Further, in this specification, the terminology used herein is for the purpose of describing a specific embodiment only and is not intended to be limiting of the present disclosure.

Further, in this specification, the singular forms "a" and "an" are intended to include the plural forms as well, unless the context clearly indicates otherwise.

Further, in this specification, it will be further understood that the terms "comprises", "comprising", "includes", and "including" specify the presence of the certain features, numbers, steps, operations, elements, and parts or combinations thereof, but do not preclude the presence or addition of one or more other features, numbers, steps, operations, elements, and parts or combinations thereof.

Further, in this specification, the term 'and/or' includes a combination of a plurality of listed items or one of the plurality of listed items. In this specification, 'A or B' may include 'A', 'B', or 'both A and B'.

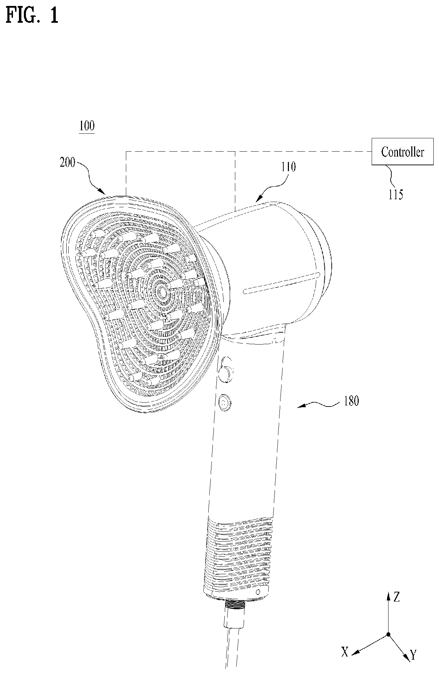

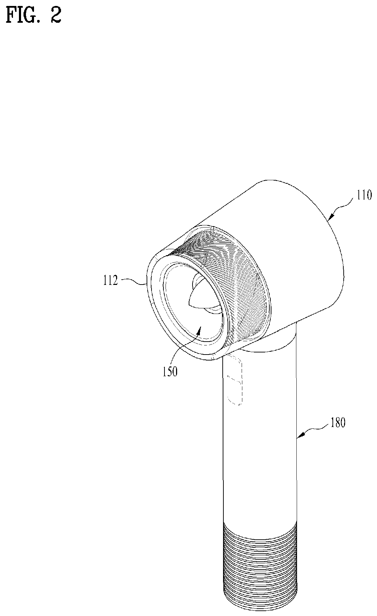

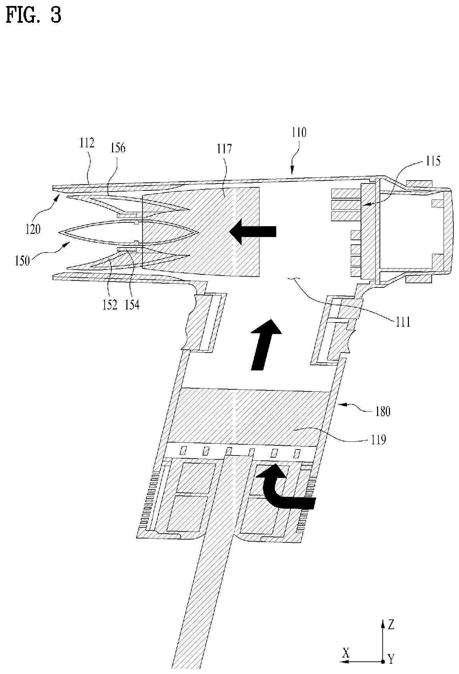

FIG. 1 is a view showing a hair dryer 100 according to an embodiment of the present invention. FIG. 2 is a view showing a state in which a diffuser 200 is separated from the hair dryer 100 shown in FIG. 1. Further, FIG. 3 is a view showing an internal cross-section of the hair dryer 100 shown in FIG. 2.

The hair dryer 100 according to an embodiment of the present invention includes a main body 110, a handle 180, and a diffuser 200 as shown in FIG. 1. In addition, as shown in FIG. 2, the main body 110 includes a gas outlet portion 150 through which gas introduced from outside is discharged.

As shown in FIG. 3, the main body 110 may include a gas flow path 111 through which the gas flows may be defined therein and the gas outlet portion 150 through which internal gas is discharged to the outside. The main body 110 may have an extended shape along a front and rear direction and may have various cross-section shapes such as circular or polygonal shapes when viewed from the front.

In the present disclosure, front, rear, left, right, top, and bottom definitions may be made centering on the main body 110. For example, referring to FIG. 2, the gas outlet portion 150 may be disposed at a front side of the main body 110, and the handle 180 may have a shape extending substantially downward from the main body 110.

The gas flowing inside the main body 110 may be introduced through a gas inlet, and the gas inlet may be disposed on the main body 110 or the handle 180. As shown in FIGS. 1 to 3, when the gas inlet is disposed on the handle 180, the gas flow path 111 may extend from the handle 180 to the main body 110, specifically, from the gas inlet to the gas outlet portion 150.

The gas may be introduced from the outside through the gas inlet disposed on the main body 110 or the handle 180, and the introduced gas may flow along the gas flow path 111 and be discharged to the outside through the gas outlet portion 150.

The handle 180 extends from the main body 110. Referring to FIGS. 1 to 3, the handle 180 extending substantially downward from the main body 110 is illustrated. The handle 180 may be integrally molded with the main body 110, or separately manufactured from the main body 110 and coupled to the main body 110.

When the handle 180 is manufactured separately from the main body 110 and coupled to the main body 110, the handle 180 may be disposed such that a longitudinal direction thereof with respect to the main body 110 is fixed or variable.

For example, the handle 180 may have a hinge coupling portion, and may be coupled to the main body 110 such that the longitudinal direction of the handle 180 is changeable, that is, the handle 180 is foldable relative to the main body 110.

The handle 180 may be a portion grabbed by a hand of a user, and thus may have a shape for improving grip convenience. The extending direction of the handle 180 may vary. However, for convenience of description below, the direction in which the handle 180 extends from the main body 110 will be described as a downward direction.

Referring to FIG. 3, the hair dryer 100 includes a fan 119 capable of flowing the gas and adjusting a speed of the discharge gas discharged through the gas outlet portion 150. The fan 119 may be disposed on the gas flow path 111 to flow the gas and may be disposed inside the main body 110 or inside the handle 180.

For example, when the gas inlet is disposed on the handle 180, the gas flow path 111 may extend from the gas inlet of the handle 180 to the gas outlet portion 150 of the main body 110, and the fan 119 may be disposed on the gas flow path 111 located in the handle 180.

In addition, a temperature adjuster 117 that may adjust a temperature of the discharge gas may be disposed inside the main body 110. The temperature adjuster 117 may be disposed in various forms and may be disposed at various positions. In FIG. 2, the temperature adjuster 117 disposed inside the main body 110 is schematically illustrated.

In addition, the temperature adjuster 117 may be disposed in various types. The temperature adjuster 117 may be in a scheme of heating the gas by providing current to a coil-shaped resistor to generate heat.

However, the resistor of the temperature adjuster 117 may not necessarily be in the shape of the coil, and may be disposed in various types, such as a thermoelement, capable of heating the gas or adjusting the temperature of the gas.

A method for operating the hair dryer 100 will be schematically described with gas flow.

First, the user manipulates a power button disposed on the main body 110 or the handle 180. When the power button is turned on, the gas is introduced into the hair dryer 100 through the gas inlet as the fan 119 is operated.

The gas introduced through the gas inlet flows along the gas flow path 111 by the fan 119 toward the gas outlet portion 150, and the discharge gas is discharged from the gas outlet portion 150 to be provided to the user. In this process, a flowing speed of the gas on the gas flow path 111 may be adjusted by the fan 119 and a temperature of the gas on the gas flow path 111 may be adjusted by the temperature adjuster 117.

In one example, referring to FIGS. 1 and 2, the hair dryer 100 may include a controller 115. The controller 115 may be connected not only to the fan 119, the temperature adjuster 117, the power button, and a manipulator, but also to a light irradiator 260, a proximity sensor 269, a moisture measurement protrusion 312, and the like of the diffuser 200 to be described later, and control the above described components.

The controller 115 may be disposed on one of the diffuser 200, the main body 110, and the handle 180. Alternatively, the controllers 115 may be respectively arranged on all of the diffuser 200, the main body 110, and the handle 180. For example, as shown in FIG. 2, the controller 115 may be disposed on the main body 110 to be signally connected to the diffuser 200, or the plurality of controllers 115 may be respectively arranged on the diffuser 200 and the main body 110.

Adjusting operating states of the fan 119 and the temperature adjuster 117 may be performed by manipulator manipulation by the user or may be automatically performed based on an operation mode preset in the controller 115.

In addition, when a distance to a target located in front of the diffuser 200 is identified to be equal to or less than a reference distance through the proximity sensor 269 of the diffuser 200, the controller 115 may control the light irradiator 260 of the diffuser 200 to irradiate light.

In addition, the controller 115 may identify an impedance of the target located in front of the diffuser 200 through the moisture measurement protrusion 312 of the diffuser 200, and determine a moisture amount of the target through the impedance.

As the moisture amount increases, the controller 115 may control the fan 119 such that the gas speed at the gas outlet portion 150 increases, control the temperature adjuster 117 such that the gas temperature increases, or control the light irradiator 260 such that a light amount of the light irradiator 260 increases.

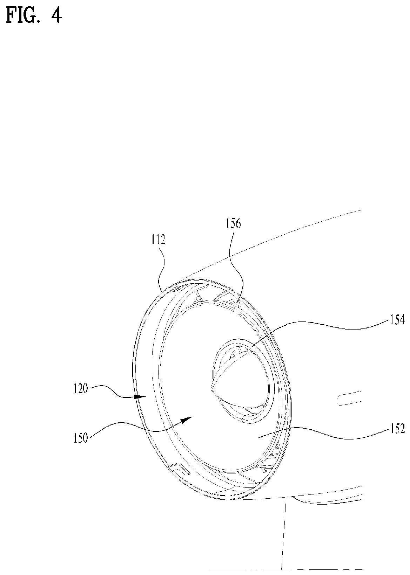

In one example, FIG. 4 shows the gas outlet portion 150 disposed on the main body 110. As shown in FIG. 1 or 3, the main body 110 may have a cross-section in an approximately circular shape and may have a length. However, the cross-section shape of the main body 110 may be varied as needed.

The gas outlet portion 150 of the hair dryer 100 according to an embodiment of the present invention will be described in detail with reference to FIG. 3.

At least a portion of the gas flow path 111 may be defined inside the main body 110, and one side of the main body 110 is opened. For example, the main body 110 may extend in the front and rear direction and a front surface thereof may be opened.

Opened one side of the main body 110 may be in communication with the gas flow path 111. In one example, the gas outlet portion 150 may be disposed on the main body 110 to shield the opened one side of the main body 110.

The opened one side of the main body 110 may correspond to an end of the gas flow path 111, and the end of the gas flow path 111 may correspond to the gas outlet portion 150. For example, the gas outlet portion 150 may be composed of the gas flow path 111 exposed through the open front surface of the main body 110 and an outer wall front end 112 of the main body 110.

In one example, as shown in FIG. 4, the gas outlet portion 150 may include a center portion 154 and side portion 156 through which the gas is discharged. The gas flowing along the gas flow path 111 may be simultaneously delivered to the center portion 154 and the side portion 156 to be discharged to the outside.

The center portion 154 and the side portion 156 may correspond to discharge holes through which the gas is discharged from the gas outlet portion 150. The center portion 154 may be defined at a central side on the cross-section of the gas outlet portion 150, and a cross-section shape thereof may be circular.

However, a shape of the center portion 154 may be a polygonal shape such as a square and the like as needed, and a size of a diameter thereof may also be varied as needed.

The side portion 156 may be defined to surround the center portion 154. For example, as shown in FIG. 4, the center portion 154 may be defined in a substantially circular shape at the center of the gas outlet portion 150, and the side portion 156 may be an opening in a shape of a ring in which the center portion 154 is defined at a center thereof.

In the present disclosure, the ring shape may have an extended shape of a closed curve shape. For example, FIG. 4 discloses the side portion 156 having a circular ring shape.

The ring shape may not necessarily be circular, and may be, for example, a polygonal ring shape such as a triangle, a square, or the like. That is, the side portion 156 may be in the circular ring or the polygonal ring shape, and FIG. 4 shows the side portion 156 having a substantially circular ring shape.

Further, the center portion 154 and the side portion 156 may be in communication with the same gas flow path 111 together. Referring to FIG. 3, there is one gas flow path 111 extending from the handle 180 inside the main body 110. The center portion 154 and the side portion 156 of the gas outlet portion 150 are in communication with the gas flow path 111 together to discharge the gas at the same time.

The discharge gas discharged from the side portion 156 may form a sense of volume for an entirety of the discharge gas discharged through the gas outlet portion 150. That is, a cross-sectional area of the entirety of the discharge gas may correspond to a size of a closed cross-section formed by the side portion 156.

However, the discharge gas of the side portion 156 may be diffused while being flowed, and a portion of the gas flow may be distributed toward a center on the cross-section where the gas is not discharged by the side portion 156, and thus, the cross-sectional area of the discharge gas may be reduced.

Accordingly, the center portion 154 is defined at a center of the side portion 156, and the phenomenon in which the discharge gas of the side portion 156 is distributed toward the center on the cross-section is suppressed by discharge gas of the center portion 154.

That is, the discharge gas of the center portion 154 flows from the center on the cross-section of the entire discharge gas of the gas outlet portion 150, and suppresses the discharge gas of the side portion 156 from being distributed toward the center during the flow process, so that it may be advantageous for the entire discharge gas to maintain an initial cross-sectional area thereof.

Accordingly, discharge gas having a large cross-sectional area may be provided to the user, and the user may perform dry using the bulky gas. For example, the entire discharge gas with the volume formed through the center portion 154 and the side portion 156 may allow the user to perform the dry in a larger area.

Further, because the center portion 154 and the side portion 156 are in communication with one gas flow path 111, the gas flow paths 111 respectively for the center portion 154 and the side portion 156 may not separately defined. Thus, it may be advantageous in terms of design and may be efficient in providing three-dimensional discharge gas to the user.

In one example, referring to FIG. 4, the gas outlet portion 150 further includes a discharge base 152 disposed on the opened one side of the main body 110. The center portion 154 may be defined at a center of the discharge base 152, and the side portion 156 may be defined between an outer circumferential surface of the discharge base 152 and an outer wall of the main body 110.

FIG. 4 illustrates the discharge base 152 coupled to the opened one side of the main body 110. The discharge base 152 may be disposed to correspond to an opened cross-sectional shape of the one side of the main body 110, but may not be limited thereto and may be formed in various shapes or materials.

For example, the discharge base 152 may be disposed to be partially different from the shape of the opened front surface of the main body 110 to determine the shape of the side portion 156, and may be molded with a material the same as or different from a material of the outer wall of the main body 110.

The discharge base 152 may constitute an entirety or a portion of one surface of the main body 110, for example, the front surface of the main body 110 as shown in FIG. 4, so that the center portion 154 may be defined at the center of the discharge base 152 and the side portion 156 may be defined between the outer circumferential surface of the discharge base 152 and the outer wall of the main body 110.

The discharge base 152 may be coupled to an opening of the main body 110 in various schemes, such as a scheme using a plurality of coupling ribs, and may be integrally molded with the main body 110.

In one example, as shown in FIG. 4, the discharge base 152 may have a shape of being indented toward an interior of the main body 110 from the side portion 156 toward the center portion 154.

A center of a front surface of the discharge base 152 may be indented toward the interior of the main body 110, so that the front surface of the discharge base 152 may form a curved surface. Accordingly, the discharge gas of the center portion 154 on the flow path of the discharge gas discharged to the gas outlet portion 150 may be discharged upstream from the discharge gas of the side portion 156.

When the discharge gas of the center portion 154 on the flow path of the entire discharge gas starts to be diffused prior to the discharge gas of the side portion 156, the cross-section of the discharge gas of the central portion 154 may be increased through the diffusion, and an effect in which the discharge gas of the center portion 154 with an increased cross-sectional area suppresses the discharge gas of the side portion 156 from being flowed or discharged toward the center may be increased.

Further, the front surface of the discharge base 152 constituting a portion of a space in which the discharge gas of the center portion 154 is diffused forms the curved surface, so that it may be advantageous in preventing formation of unnecessary turbulence. A curvature of the curved surface formed by the front surface of the base portion may be variously set as necessary.

In one example, an embodiment of the present disclosure may further include a guide cone disposed at a center of the center portion 154 and guiding the flow of the gas discharged through the center portion 154. The gas may be discharged between an inner surface of the center portion 154 and the guide cone.

FIG. 4 illustrates the guide cone disposed at the center of the center portion 154. As the guide cone is disposed, the discharge gas of the center portion 154 is discharged into a space between the inner surface of the center portion 154 and an outer surface of the guide cone.

When the guide cone is disposed at the center of the center portion 154, the center portion 154 may correspond to a ring-shaped discharge hole. That is, the discharge gas of the center portion 154 may have a ring-shaped cross-section and may be discharged from the center portion 154.

As described above, the discharge gas of the center portion 154 may contribute to suppressing the reduction of the cross-sectional area resulted from the discharge gas of the side portion 156 that flows toward the center in the flow process. In addition, an embodiment of the present disclosure may increase a level at which the discharge gas of the center portion 154 diffuses outward from the cross-section by disposing the guide cone at the center of the center portion 154.

When the cross-sectional area of the discharge gas of the center portion 154 is increased as the guide cone is disposed, the effect of suppressing the phenomenon in which the discharge gas of the side portion 156 flows inward of the cross-section may be increased.

In one example, in the guide cone, a rear end protruding toward the gas flow path 111 and a front end protruding in a discharge direction of the gas of the center portion 154 may respectively have conical shapes. The conical shape means a shape in which a cross-section has a circular shape and a diameter of the circle gradually decreases as a length increases.

However, in the conical shape, the circular shape may include a shape other than a definite circular shape such as an ellipse and the like, and the reduction in the diameter may not necessarily be constant, for example, a diameter reduction rate may gradually increase or gradually decrease.

Further, as the front end of the guide cone protrudes in the conical shape, an effect in which the discharge gas of the center portion 154 is concentrated toward the rim of the center portion 154 increases. Thus, the effect of suppressing the discharge gas of the side portion 156 from flowing toward the discharge gas of the center portion 154 may be further increased.

An outer circumferential surface of the guide cone may have a shape corresponding to an inner circumferential surface of the center portion 154, and a separation distance between the outer circumferential surface of the guide cone and the inner circumferential surface of the center portion 154 may be varied as needed.

Further, the guide cone may be made of a material the same as or different from the material of the discharge base 152, and a curvature of the outer surface thereof may be variously designed as needed.

In one example, the gas outlet portion 150 may further include a discharge guide ring. The discharge guide ring may be disposed on the inner surface of the center portion 154 and protrude in the discharge direction of the gas of the center portion 154 to guide the gas flow together with the guide cone. FIG. 4 illustrates that the guide cone and the discharge guide ring are arranged in the center portion 154.

The discharge guide ring may have a ring shape extending along the rim of the center portion 154, and may be integrally molded with the discharge base 152 or molded separately from the discharge base 152 to be coupled to the inner circumferential surface of the center portion 154.

The discharge guide ring may protrude outward from the center portion 154 or the discharge base 152 based on the gas discharge direction. The flow of the discharge gas of the center portion 154 may be concentrated between the guide cone and the discharge guide ring by the guide cone and the discharge guide ring protruding from the center portion 154.

A protruding end of the discharge guide ring may have a curved shape to facilitate the gas flow. A diameter of the discharge guide ring may be different for each portion, and the shape thereof may also be varied as needed.

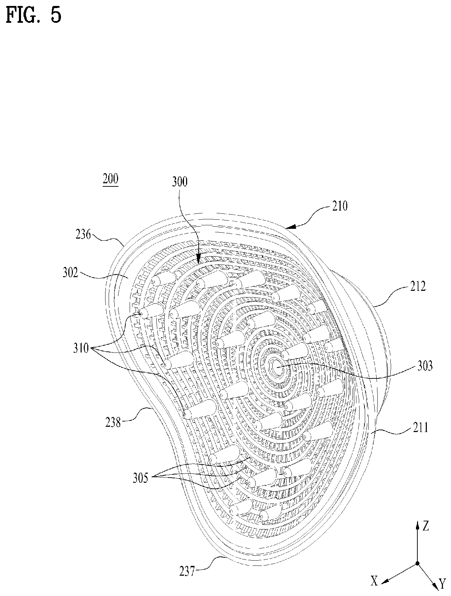

In one example, FIG. 5 shows the diffuser 200 according to an embodiment of the present invention. The diffuser 200 is removably coupled to the main body 110, so that the gas discharged from the gas outlet portion 150 is introduced into the diffuser 200 and the gas introduced into the diffuser 200 is discharged to the outside.

The hair dryer 100 according to an embodiment of the present invention may include the diffuser 200 as shown in FIG. 1, and the diffuser 200 may be removably coupled to the main body 110 of the hair dryer 100.

The diffuser 200 may be disposed such that gas discharged from the gas outlet portion 150 of the main body 110 flows into the diffuser 200. The diffuser 200 may be coupled to the main body 110 such that a rear side thereof covers the gas outlet portion 150, and the gas discharged from the gas outlet portion 150 may flow into the diffuser 200 through a gas inlet hole 215 defined at a rear side of the diffuser 200.

The user may selectively use the diffuser 200 for scalp or hair management. For example, the user may use the diffuser 200 including a massage protrusion 310 and a light irradiator 260, which will be described later, for scalp care, or may use the diffuser 200 at which a flow cross-sectional area of the gas is increased as needed in a hair drying step,

The rear side of the diffuser 200 may be coupled to the front end 112 of the main body 110.

A first coupling portion 120 may be disposed at the front end 112 of the main body 110, and a second coupling portion 220 coupled to the first coupling portion 120 may be disposed at the rear side of the diffuser 200.

A coupling scheme between the diffuser 200 and the main body 110 may vary. The diffuser 200 may be coupled to the main body 110 in a scheme such as screw coupling, fitting coupling, magnetic coupling, sliding coupling, and the like to receive the gas from the main body 110.

An embodiment of the present disclosure may improve ease of use of the user as the diffuser 200 is disposed to be removable from the main body 110. For example, the user may use the hair dryer 100 by removing the diffuser 200 when the user is desired to use the gas discharged from the gas outlet portion 150 of the main body 110 as it is. Further, the user may use the hair dryer 100 coupled to the diffuser 200 when the user wants a more diffused flow cross-sectional area.

The diffuser 200 may include a diffusing case 210 and a discharge cover 300, and the diffusing case 210 and a discharge cover 300 may form an exterior of the diffuser 200.

An inner diameter of the diffuser 200 increases the inner diameter in a forward direction. That is, the diffusing case 210 of the diffuser 200 is disposed such that an internal cross-sectional area thereof viewed from the front increases from a rear side 212 to a front side 211.

Accordingly, the gas delivered from the gas outlet portion 150 may be provided to the user in a state in which the flow cross-sectional area thereof is increased as the gas speed is reduced in the forward direction of the diffuser 200. The user may use the diffuser 200 for natural drying, styling, and the like of the hair.

The front side 211 of the diffusing case 210 may be opened to define an open front surface. An entirety or a portion of the front surface of the diffuser 200, that is, the diffusing case 210, may define the open surface.

The gas present inside the diffuser 200 may be discharged to the outside through the open surface of the diffusing case 210. That is, the gas inside the diffuser 200 may be provided to the user while being discharged forward through the front side 211 of the diffusing case 210.

In the diffuser 200, the open surface defined in the front side 211 of the diffusing case 210 may be exposed to the outside as it is, or the discharge cover 300 may be provided coupled to the open surface.

FIG. 5 shows a state in which the open surface is present in the front side 211 of the diffusing case 210 according to an embodiment of the present disclosure and the discharge cover 300 is coupled to the open surface.

The discharge cover 300 coupled to the open surface defined in the front side 211 of the diffusing case 210 may include a gas discharge hole 305 defined therein through which the gas may be discharged. The discharge cover 300 has a shape corresponding to the open surface of the diffusing case 210 and may be coupled to the diffusing case 210 to be located on the open surface.

A plurality of gas discharge holes 305 may be defined and may be spaced apart from each other in the front surface of the discharge cover 300. FIG. 5 shows the discharge cover 300 in which the plurality of gas discharge holes 305 are uniformly distributed and arranged in the front surface.

Accordingly, in the diffuser, the gas may be discharged through the entirety of the front surface of the discharge cover 300 and the user may receive the gas that is discharged forward of the discharge cover 300 and uniformly dispersed.

The discharge cover 300 may be disposed such that an edge 302 located on the outermost side along a radial direction of the diffuser 200 is in close contact with the diffusing case 210. That is, the diffusing case 210 may have a front circumferential portion 236 surrounding the open surface in the front side 211, and the discharge cover 300 may be disposed such that the edge 302 has a shape corresponding to the front circumferential portion 236 and in contact with the front circumferential portion 236.

As shown in FIG. 5, the diffuser 200 according to an embodiment of the present disclosure may have a first portion 237 and a second portion 238 on the front circumferential portion 236 of the diffusing case 210. The first portion 237 and the second portion 238 may be arranged with different distances from the rear side 212 of the diffusing case 210, for example, the gas inlet hole 215 of the diffusing case 210.

In addition, the discharge cover 300 may be disposed such that the edge 302 is molded to correspond to shapes of the first portion 237 and the second portion 238 to be in close contact with the front circumferential portion 236 of the diffusing case 210.

The front circumferential portion 236 of the diffusing case 210 and the edge 302 of the discharge cover 300 may be designed to fit a head of the user with an arbitrary curved surface while respectively having curvatures and having different lengths protruding forward along an outer circumferential direction of the diffuser 200. Accordingly, a rate of adhesion with the scalp or the hair of the user may be efficiently increased to minimize a space between the head of the user and the diffuser 200.

Accordingly, an amount of the gas discharged forward of the discharge cover 300 or the light provided by the light irradiator 260 may be efficiently increased.

An ergonomic design is made through the front circumferential portion 236 of the diffusing case 210 and the edge 302 of the discharge cover 300 respectively arranged to form curves when viewed from the side as described above. In this case, the curvatures and the like of the front circumferential portion 236 and the edge 302 may be designed based on a standard head that is statistically determined.

For example, an embodiment of the present disclosure may define a R127 curvature design from a shape of the standard head, and design the shapes of the front circumferential portion 236 and the edge 302 to correspond thereto.

In one example, a proximity sensor 269 may be disposed inside the diffusing case 210 to improve ease of use and efficiency of the diffuser 200, and an open region 303 may be defined in the discharge cover 300 such that a distance measurement accuracy of the proximity sensor 269 for the target in front of the diffuser 200, for example, the hair or the scalp of the user may be improved.

That is, the proximity sensor 269 may be in various schemes such as pressure, ultrasound, infrared, and the like to measure the distance to the target in front of the proximity sensor 269, and a region of the discharge cover 300 in front of the proximity sensor 269 may be opened to define the open region 303.

In one example, FIG. 5 shows the discharge cover 300 on which a plurality of massage protrusions 310 are arranged. The massage protrusion 310 may have a pillar shape protruding forward of the diffuser 200 and may press the scalp of the user to provide a massage effect.

A cross-section shape, a protruding length, an arrangement form, and the like of the massage protrusion 310 may be variously determined in terms of a design. An embodiment of the present disclosure provides the user with scalp massage through the massage protrusion 310 while providing the gas diffused through a front surface of the discharge cover 300 to the user, thereby providing the improved ease of use and the scalp and hair care effects.

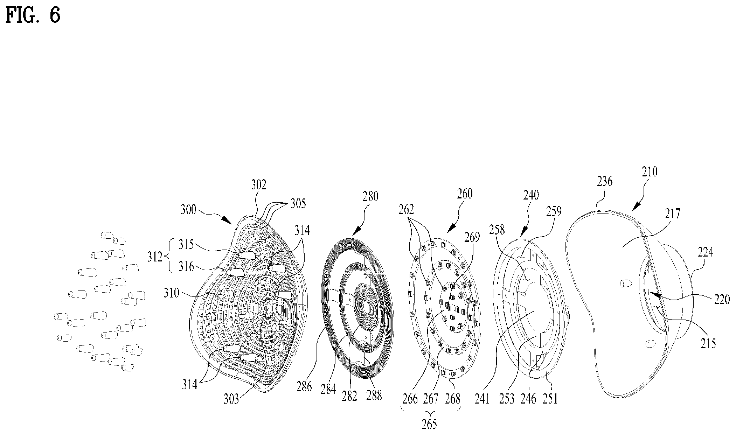

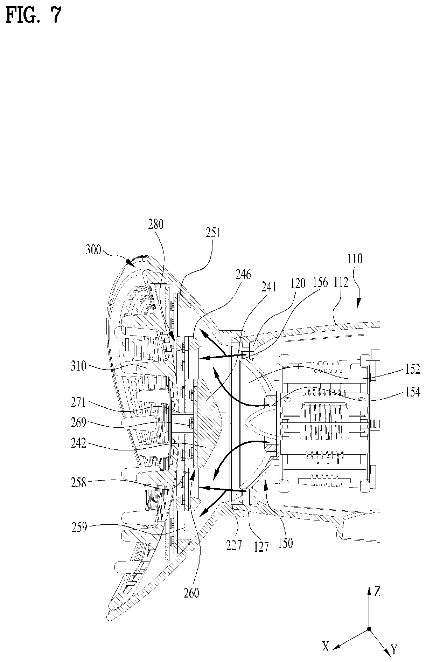

FIG. 6 is a view showing an exploded view of the diffuser 200 according to an embodiment of the present invention, and FIG. 7 is a view showing an internal cross-section of the diffuser 200 according to an embodiment of the present invention.

Referring to FIGS. 6 and 7, the diffuser 200 may include the diffusing case 210, a guide frame 240, the light irradiator 260, a light diffusion frame 280, and the discharge cover 300.

In the diffusing case 210, the rear side 212 may be coupled with the main body 110, and the open surface may be defined in the front side 211. The inner diameter of the diffusing case 210 increases from the rear side 212 to the front side 211, so that the inside gas may be diffused and discharged to the outside.

That is, the gas discharged through the gas outlet portion 150 of the main body 110 may be provided to the user in a state in which the flow cross-sectional area thereof is increased as the gas is flowing in the diffusing case 210.

FIGS. 6 and 7 show the diffusing case 210 in which the inner diameter thereof increases from the rear side 212 to the front side 211 and accordingly an outer diameter thereof increases in the same manner.

The gas inlet hole 215 may be defined in the rear side 212 of the diffusing case 210. When the diffusing case 210 is coupled to the main body 110, the gas inlet hole 215 is positioned to face the gas outlet portion 150. Further, the gas discharged from the gas outlet portion 150 may be introduced into the diffusing case 210 through the gas inlet hole 215.

The gas inlet hole 215 may be located at a center of the rear side 212 of the diffusing case 210 when viewed from the rear, and a cross-section shape of the gas inlet hole 215 may correspond to the gas outlet portion 150.

For example, the gas inlet hole 215 is defined to have an inner diameter larger than the side portion 156 of the gas outlet portion 150, so that the gas discharged from the gas outlet portion 150 may be completely introduced into the diffusing case 210 through the gas inlet hole 215.

The second coupling portion 220 coupled to the main body 110 may be disposed on the rear side 212 of the diffusing case 210. The diffusing case 210 may include a rear circumferential portion 217 surrounding the gas inlet hole 215 in the rear side 212, and the second coupling portion 220 may be disposed at the rear circumferential portion 217.

The second coupling portion 220 may further include a coupling sleeve 224. The coupling sleeve 224 may extend rearward from the rear circumferential portion 217. The coupling sleeve 224 may be disposed to outwardly surround the front end 112 of the main body 110.

The second coupling portion 220 may have a second magnetic fastening portion 227 embedded in the rear circumferential portion 217 or located inside the rear circumferential portion 217, and may include a power receiver disposed on an inner surface and the like of the coupling sleeve 224.

In addition, the first coupling portion 120 may be disposed at the front end 112 of the main body 110, may have a first magnetic fastening portion 127 embedded inside the outer wall of the front end 112 or located inside the outer wall, and may include a power transmitter disposed on an outer surface and the like of the outer wall of the front end 112.

The first coupling portion 120 is coupled to the second coupling portion 220. At least one of the first magnetic fastening portion 127 and the second magnetic fastening portion 227 may include a magnetic force generator, so that the first magnetic fastening portion 127 and the second magnetic fastening portion 227 may be magnetically coupled to each other. The magnetic coupling means a scheme of mutual coupling through a magnetic force generated from the magnetic force generator such as a magnet and an electromagnet.

The power transmitter may supply power to the power receiver in contact or connection with the power receiver. The power receiver may be connected to a component inside the diffuser 200, for example, the light irradiator 260 and the like to supply power thereto.

The open surface surrounded by the front circumferential portion 236 may be defined in the front side 211 of the diffusing case 210, and the gas inside the diffusing case 210 may be discharged forward of the diffuser 200 through the open surface in the front side 211.

In one example, the guide frame 240 is disposed inside the diffusing case 210. The guide frame 240 is disposed to guide the flow of the gas introduced through the gas inlet hole 215.

The guide frame 240 is disposed to face the gas inlet hole 215 of the diffusing case 210. The guide frame 240 may have a diffusion portion 241 at a center thereof, a first guide 246 disposed radially outward of the diffusion portion 241, and a second guide 251 disposed radially outward of the first guide 246.

The guide frame 240 may include a guide connector 253 extending along the radial direction of the diffuser 200, and the guide connector 253 may connect the diffusion portion 241, the first guide 246, and the second guide 251 to each other.

The diffusion portion 241 of the guide frame 240 is disposed to face the gas inlet hole 215 to diffuse the gas introduced through the gas inlet hole 215 outward in the radial direction. That is, the flow cross-sectional area of the gas introduced through the gas inlet hole 215 may be increased by the diffusion portion 241.

In the gas outlet portion 150 having the center portion 154 and the side portion 156, a flow direction of the gas discharged from the center portion 154 may be changed by the diffusion portion 241. That is, the diffusion portion 241 may have a larger diameter than the center portion 154 and diffuse the gas provided from the center portion 154 outward in the radial direction.

The first guide 246 may have a ring shape, and the diffusion portion 241 may be located at a center of the first guide 246. The diffusion portion 241 may have a circular cross-section, and may be outwardly spaced apart from the diffusion portion 241 while being concentric with the diffusion portion 241 of the first guide 246.

A first flow path 258 may be located between the first guide 246 and the diffusion portion 241. That is, the first guide 246 may be spaced apart from the diffusion portion 241 to define the first flow path 258 between the first guide 246 and the diffusion portion 241. The gas diffused through the diffusion portion 241 may flow through the first flow path 258.

The second guide 251 may have a ring shape corresponding to the first guide 246, and the diffusion portion 241 and the first guide 246 may be located at a center of the second guide 251. That is, the second guide 251 may be concentric with the diffusion portion 241 and the first guide 246 and may be spaced apart from the first guide 246.

That is, an inner diameter of the first guide 246 may be larger than the diameter of the diffusion portion 241, and an inner diameter of the second guide 251 may be larger than an outer diameter of the first guide 246. Accordingly, the first flow path 258 may be defined between the diffusion portion 241 and the first guide 246, and a second flow path 259 may be defined between the first guide 246 and the second guide 251.

The gas diffused by the diffusion portion 241 may flow through the first flow path 258 and the second flow path 259. An outer diameter of the second flow path 259 may be larger than the diameter of the gas inlet hole 215, so that the gas introduced through the gas inlet hole 215 may be diffused by the diffusion portion 241 and flow with a larger flow cross-section.

The light irradiator 260 may be located in front of the guide frame 240. The light irradiator 260 may be installed on a front surface of the guide frame 240. The light irradiator 260 may have a plurality of light emitters 262 arranged on a circuit board 265. The circuit board 265 may include a plurality of circuit boards separated from each other, and the plurality of boards may be respectively arranged on the diffusion portion 241, the first guide 246, and the second guide 251 of the guide frame 240.

The light irradiator 260 may include the plurality of circuit boards 265, and the plurality of circuit boards 265 may respectively include a central board 266, a first board 267, and a second board 268.

The central board 266 may have a cross-section shape corresponding to the diffusion portion 241. For example, the diffusion portion 241 may have the circular cross-section, and the central board 266 may have a circular cross-section in the same manner as the diffusion portion 241, may be disposed on a front surface of the diffusion portion 241, and may include the plurality of light emitters 262.

The first board 267 may have a shape corresponding to the first guide 246. For example, the first guide 246 may have the ring shape, and the first board 267 may have a ring shape in the same manner as the first guide 246, may be disposed on a front surface of the first guide 246, and may include the plurality of light emitters 262.

The second board 268 may have a shape corresponding to the second guide 251. For example, the second guide 251 may have the ring shape, and the second board 268 may have a ring shape in the same manner as the second guide 251, may be disposed on a front surface of the second guide 251, and may include the plurality of light emitters 262.

The central board 266, the first board 267, and the second board 268 may be arranged to be concentric like the guide frame 240. The first board 267 may be outwardly spaced apart from the central board 266, and the second board 268 may be outwardly spaced apart from the first board 267.

That is, an inner diameter of the first board 267 may be larger than a diameter of the central board 266, and an inner diameter of the second board 268 may be larger than an outer diameter of the first board 267. Therefore, like the guide frame 240, the first flow path 258 may be located between the central board 266 and the first board 267, and the second flow path 259 may be located between the first board 267 and the second board 268.

The light irradiator 260 may irradiate light toward the front side 211 of the diffusing case 210 through the plurality of light emitters 262. The light irradiated from the light irradiator 260 may be provided to a location ahead of the diffuser 200 through the front side 211 of the diffusing case 210.

For example, the light irradiated from the light irradiator 260 may be provided to the location ahead of the diffuser 200 by passing through the open surface of the diffusing case 210, passing through the gas discharge hole 305 of the discharge cover 300, passing through the discharge cover 300, or passing through the massage protrusion 310 of the discharge cover 300.

As the light is irradiated to the location ahead of the diffuser 200, the diffuser 200 may perform user's hair or scalp care. The light irradiated from the light irradiator 260 may contribute to improving scalp and hair health while drying the user's scalp or hair or providing heat to the user's scalp or hair.

The proximity sensor 269 may be disposed on the circuit board 265 of the light irradiator 260. FIG. 6 shows a state in which the proximity sensor 269 is disposed on the central board 266 of the light irradiator 260 according to an embodiment of the present disclosure.

The proximity sensor 269 may be disposed at a center of the central board 266. The proximity sensor 269 may be disposed to measure the separation distance from the target positioned in front of the proximity sensor 269. The controller 115 may be disposed to control the light irradiator 260 based on the separation distance between the proximity sensor 269 and the front target measured by the proximity sensor 269.

For example, when the separation distance from the target measured by the proximity sensor 269 is equal to or less than a reference distance, the controller 115 may control the light irradiator 260 such that the light irradiator 260 irradiates the light forward. The reference distance may be predetermined in terms of a design or control.

However, the light irradiator 260 may be operated through a physical switch that is operated when the separation distance measured by the proximity sensor 269 is equal to or less than the reference distance.

As the proximity sensor 269 is used, the light irradiator 260 is operated when the separation distance from the target in front of the diffuser 200, for example, the scalp or the hair of the user is equal to or less than the reference distance, thereby improving ease of use and an operation efficiency.

The proximity sensor 269 may be disposed in various types. For example, the proximity sensor 269 may be a pressure sensor that detects whether a pressing force is applied from the user's scalp or hair, or a photosensitive sensor that measures a level at which an amount of sensed light decreases as the separation distance from the scalp or the hair decreases.

In addition, the proximity sensor 269 may be an IR sensor that measures an infrared ray transmitted from the front target, that is, the user's scalp or hair to measure the separation distance from the scalp or the hair. In this case, the proximity sensor 269 may be disposed to irradiate the infrared ray forward.

In one example, the light diffusion frame 280 may be located in front of the light irradiator 260. The light diffusion frame 280 may be installed on a front surface of the light irradiator 260. That is, the light diffusion frame 280 may be disposed to forwardly cover the light irradiator 260.

The light diffusion frame 280 may include a central light diffusion portion 282, a first light diffusion portion 284 and a second light diffusion portion 286. The light diffusion frame 280 may further include a light diffusion connector 288 for connecting the central light diffusion portion 282, the first light diffusion portion 284, and the second light diffusion portion 286 to each other.

The central light diffusion portion 282 may have a cross-section shape corresponding to the central board 266. For example, the central board 266 may have the circular cross-section, and the central light diffusion portion 282 may have a circular cross-section in the same manner as the central board 266 and may cover the front surface of the diffusion portion 241.

The first light diffusion portion 284 may have a shape corresponding to the first board 267. For example, the first board 267 may have the ring shape, and the first light diffusion portion 284 may have a ring shape in the same manner as the first board 267 and may cover the front surface of the first board 267.

The second light diffusion portion 286 may have a shape corresponding to the second board 268. For example, the second board 268 may have the ring shape, and the second light diffusion portion 286 may have a ring shape in the same manner as the second board 268 and may cover the front surface of the second board 268.

The central light diffusion portion 282, the first light diffusion portion 284, and the second light diffusion portion 286 may be arranged to be concentric like the guide frame 240 and the light irradiator 260. The first light diffusion portion 284 may be outwardly spaced apart from the central light diffusion portion 282, and the second light diffusion portion 286 may be outwardly spaced apart from the first light diffusion portion 284.

That is, an inner diameter of the first light diffusion portion 284 may be larger than a diameter of the central light diffusion portion 282, and an inner diameter of the second light diffusion portion 286 may be larger than an outer diameter of the first light diffusion portion 284. Accordingly, like the guide frame 240, the first flow path 258 may be located between the central light diffusion portion 282 and the first light diffusion portion 284, and the second flow path 259 may be located between the first light diffusion portion 284 and the second light diffusion portion 286.

That is, the diffuser 200 may be disposed in a shape in which the first flow path 258 and the second flow path 259 are extended in the front and rear direction through the guide frame 240, the light irradiator 260, and the light diffusion frame 280.

The light diffusion connector 288 may be disposed in a shape corresponding to the guide connector 253. For example, the guide connector 253 and the light diffusion connector 288 may have an extended shape along the radial direction of the diffuser 200.

The light diffusion connector 288 may be located in front of the guide connector 253. The light diffusion frame 280 may be fixed inside the diffusing case 210 as the light diffusion frame 280 is fastened to the guide connector 253.

An embodiment of the present invention is advantageous in terms of a design and structurally stable in that, in a state in which the guide frame 240 is constituted by a plurality of components, the plurality of components are able to be handled as a single component through the guide connector 253.

In addition, an embodiment of the present invention is advantageous in terms of the design and structurally stable in that, in a state in which the light diffusion frame 280 is constituted by a plurality of components, the plurality of components are able to be handled as a single component through the light diffusion connector 288.

Furthermore, the light diffusion connector 288 of the light diffusion frame 280 is coupled to the guide connector 253 of the guide frame 240, so that all of the central light diffusion portion 282, the first light diffusion portion 284, and the second light diffusion portion 286 may be stably fixed, which is advantageous in terms of coupling.

The light diffusion frame 280 may be made of a material through which light is transmitted. For example, the light diffusion frame 280 may be made of a transparent or translucent material. The light irradiated from the light irradiator 260 may be scattered and diffused while passing through the light diffusion frame 280.

In the diffuser 200, the light diffusion frame 280 is disposed in front of the light irradiator 260, so that the light irradiated from the light irradiator 260 may be provided to the user while being scattered and diffused and being uniformly dispersed in a larger area.

A treatment for the diffusion or the scattering of the light may be performed on a front surface or a rear surface of the light diffusion frame 280. For example, etching may be performed or a pattern through laser processing and the like may be formed on a surface of the light diffusion frame 280.

In one example, the central light diffusion portion 282 is disposed to shield the front surface of the central board 266, and a portion of the central light diffusion portion 282 in front of the proximity sensor 269 may be opened such that the measurement of the separation distance from the target in front of the diffuser 200 by the proximity sensor 269 disposed on the central board 266 is easy.

FIG. 6 shows a state in which the proximity sensor 269 is disposed at the center of the central board 266 and the central light diffusion portion 282 has a hole defined at a center thereof to expose the proximity sensor 269 forwardly.

The discharge cover 300 may be disposed to shield the open surface defined in the front side 211 of the diffusing case 210 in which the guide frame 240, the light irradiator 260, and the light diffusion frame 280 are embedded. The plurality of gas discharge holes 305 are defined in the discharge cover 300, so that the gas may be discharged and the light may be irradiated forward.

The discharge cover 300 may be disposed such that the edge 302 has a curvature to correspond to the front circumferential portion 236 of the diffusing case 210 when viewed from the side and is indented rearwards centerwardly when viewed from the front.

That is, a front surface of the discharge cover 300 may form a curved surface that is indented rearwards centerwardly, so that the discharge cover 300 may have a shape corresponding to the head of the user and may be optimized to provide the massage effect through the massage protrusion 310 while providing the gas and the light to the user.

The plurality of massage protrusions 310 protruding or extending forward on the front surface of the discharge cover 300 may be arranged, and a contact portion may be disposed on the surface of the discharge cover 300 such that a sense of touch with the scalp or the hair of the user may be improved and damage to the scalp and the hair may be minimized. The contact portion may be made of a material such as silicon and the like.

Some of the plurality of massage protrusions 310 may correspond to the moisture measurement protrusions 312. That is, in the diffuser 200, the plurality of massage protrusions 310 may include the moisture measurement protrusions 312.

The moisture measurement protrusion 312 may be disposed to measure a moisture amount of the scalp or the hair of the user. A pair of the moisture measurement protrusions 312 may be arranged to measure an impedance, that is, bioelectrical impedance through an electric field formed therebetween.

The moisture measurement protrusion 312 may be connected to the controller 115. Further, the controller 115 may determine the impedance using a voltage, a current, a resistance, and the like, which are identified through the moisture measurement protrusion 312, determine the moisture amount of the scalp or the hair of the user based on the determined impedance, and control an operation of the fan 119, the temperature adjuster 117, or the light irradiator 260 based on the determined moisture amount.

For example, the controller 115 may control the fan 119 such that the gas speed increases as the moisture amount of the scalp or the hair of the user increases, control the temperature adjuster 117 such that the gas temperature increases, or control the light irradiator 260 such that the light amount increases.

The pair of moisture measurement protrusions 312 may be arranged. The moisture measurement protrusions 312 may include a first moisture measurement protrusion 315 electrically having a first pole and a second moisture measurement protrusion 316 having a second pole opposite to the first pole.

The controller 115 may determine the impedance and the moisture amount through the electric field formed between the first moisture measurement protrusion 315 and the second moisture measurement protrusion 316.

A plurality of pairs of moisture measurement protrusions 312, each of which is constituted by the first moisture measurement protrusion 315 and the second moisture measurement protrusion 316, may be arranged. One pair of moisture measurement protrusions 312 may be disposed to be spaced apart from another pair of moisture measurement protrusions, and different massage protrusions 310 may be positioned therebetween.

In one example, the open region 303 may be defined at the center of the discharge cover 300. The proximity sensor 269 may be exposed forward through the hole defined in the light diffusion frame 280 and the open region 303 of the discharge cover 300, and may wholly measure the separation distance from the target in front of the diffuser 200. A protection member that protects the proximity sensor 269 and allows the infrared ray or the like to pass straight therethrough may be disposed in front of the proximity sensor 269.

FIG. 7 shows an internal cross-section of the diffuser 200 and a state in which the diffuser 200 is coupled to the main body 110 to receive the gas from the gas outlet portion 150.

Referring to FIG. 7, the first coupling portion 120 of the main body 110 may include the first magnetic fastening portion 127 and the second coupling portion 220 of the diffuser 200 may include the second magnetic fastening portion 227.

The diffuser 200 may be coupled to the front end 112 of the main body 110 through the magnetic coupling between the first magnetic fastening portion 127 and the second magnetic fastening portion 227. The first coupling portion 120 may further include a hook fastener and the second coupling portion 220 may further include a hook fastened to the hook fastener, so that a coupling stability between the diffuser 200 and the main body 110 may be enhanced.

Hereinafter, the flow of the gas discharged from the gas outlet portion 150 will be described with reference to FIG. 7.

In the gas outlet portion 150, the gas is discharged from the center portion 154 and the side portion 156. The gas inlet hole 215 of the diffusing case 210 is defined to have a diameter equal to or larger than that of the side portion 156 and face the gas outlet portion 150, so that the gas discharged from the center portion 154 and the side portion 156 may be introduced into the inlet hole 215.

The guide frame 240 may be disposed inside the diffusing case 210 to face the gas outlet portion 150. Specifically, the diffusion portion 241 of the guide frame 240 may be positioned to face the center portion 154 of the gas outlet portion 150.

The gas discharged from the center portion 154 flows toward the diffusion portion 241. As the diffusion portion 241 has a diameter larger than that of the center portion 154, the gas discharged from the center portion 154 may be diffused along the radial direction of the diffuser 200.

The diffusion portion 241 may have a diffusion protrusion 242 on a rear surface thereof facing the center portion 154, and the diffusion effect of the gas discharged from the center portion 154 may be improved by the diffusion protrusion 242. The diffusion protrusion 242 may be disposed to increase in rearwardly protruding height toward a center on the cross-section when viewed from the rear.

At least a portion of the gas discharged from the center portion 154 may flow along the first flow path 258 defined between the diffusion portion 241 and the first guide 246 in the guide frame 240 by the diffusion portion 241 and the diffusion protrusion 242.

In one example, the gas discharged from the side portion 156 may have a flow form outwardly surrounding the gas discharged from the center portion 154, and the gas discharged from the side portion 156 may also diffuse outward along the radial direction of the diffuser 200 as the gas of the center portion 154 is diffused by the diffusion portion 241.

Therefore, at least a portion of the gas discharged from the side portion 156 and at least a portion of the gas discharged from the center portion 154 may flow along the second flow path 259 defined between the first guide 246 and the second guide 251 in the guide frame 240.

Despite a design feature that the inner diameter of the diffuser 200 increases forwardly, the discharging of the gas discharged from the center portion 154 and the side portion 156 in the forward direction while being maintained in a specific form may be effectively suppressed through the guide frame 240.

Furthermore, the diffuser 200 allows the gas discharged from the center portion 154 and the side portion 156 to be effectively dispersed and diffused with a larger flow cross-sectional area while preventing the flow of the gas from being maintained in the specific form.

In one example, the light irradiator 260 and the light diffusion frame 280 may be arranged in front of the guide frame 240 inside the diffusing case 210. The light irradiator 260 and the light diffusion frame 280 may be coupled with the guide frame 240 and thus may be handled as a single component, so that a structure that is excellent in a space utilization and is advantageous in design may be implemented.

In addition, the light irradiator 260 and the light diffusion frame 280 may define the first flow path 258 and the second flow path 259 together with the guide frame 240.As the structure in which the light irradiator 260 and the light diffusion frame 280 define the first flow path 258 and the second flow path 259 together with the guide frame 240 is achieved, the flow of the gas formed by the guide frame 240 may be effectively maintained and the gas may be discharged forwardly of the diffuser 200 through the light irradiator 260 and the light diffusion frame 280.

In one example, in the light irradiator 260, the first board 267 may be positioned forwardly of the central board 266, and the second board 268 may be positioned forwardly of the first board 267. Accordingly, the plurality of light emitters 262 arranged in the light irradiator 260 may be arranged to form a spherical surface that is substantially indented rearward.

Accordingly, the plurality of light emitters 262 may be arranged in a form in which a distance from a center of the light irradiator 260 along the radial direction increases forwardly. Such arrangement of the light emitters 262 may correspond to the shape of the front surface of the discharge cover 300 indented rearward.

That is, the plurality of light emitters 262 arranged on the light irradiator 260 are arranged to form the curved surface to correspond to the user's head having the curvature, so that a uniform amount of light may be provided to the user's scalp and hair.

Like the light irradiator 260, the guide frame 240 may be disposed such that the first guide 246 is positioned forwardly of the diffusion portion 241 and the second guide 251 is positioned forwardly of the first guide 246.

Accordingly, the first board 267 disposed on the front surface of the first guide 246 may be positioned forwardly of the central board 266 disposed on the front surface of the diffusion portion 241, and the second board 268 disposed on the front surface of the second guide 251 may be positioned forwardly of the first board 267.

Like the light irradiator 260, in the light diffusion frame 280, the first light diffusion portion 284 may be positioned forwardly of the central light diffusion portion 282 and the second light diffusion portion 286 may be positioned forwardly of the first light diffusion portion 284. Accordingly, a distance between the light diffusion frame 280 and the light irradiator 260 may be kept constant, and uniform dispersion and scattering of the light may be induced.

In addition, in the guide frame 240, as the second guide 251 is positioned forwardly of the first guide 246 and the first guide 246 is positioned forwardly of the diffusion portion 241, a space in which the gas introduced from the gas inlet hole 215 is diffused in the radial direction may be secured and the gas may be smoothly introduced into the first flow path 258 and the second flow path 259.

FIG. 7 shows the guide frame 240, the light irradiator 260, and the light diffusion frame 280 protruding forward in a direction away from centers thereof, according to an embodiment of the present invention.

In one example, FIG. 7 shows a light blocking portion 271 surrounding the proximity sensor 269. The light blocking portion 271 may be disposed to surround the proximity sensor 269 along the circumferential direction of the diffuser 200, thereby preventing a situation in which the light emitter 262 around the proximity sensor 269 affects the proximity sensor 269.

In addition, the light blocking portion 271 may be opened forward to prevent structural interference from occurring in a measurement of the separation distance between the diffuser 200 and the front target by the proximity sensor 269. For example, when the proximity sensor 269 measures the infrared ray transmitted from the target, the light blocking portion 271 is opened forward to allow the infrared ray transmitted from the target to be completely provided to the proximity sensor 269.

The light blocking portion 271 may be formed in a hollow cylindrical shape. The proximity sensor 269 may be located inside the light blocking portion 271. The light blocking portion 271 may have a shape extending from the central board 266 to the discharge cover 300.

The light blocking portion 271 may be disposed to extend rearward from the discharge cover 300, or may be formed integrally with the discharge cover 300 or integrally with the central board 266. The light blocking portion 271 may be manufactured separately from the discharge cover 300 and the central board 266, and may be coupled or connected to the discharge cover 300 and/or the central board 266.

In one example, as described above, the hair dryer 100 according to an embodiment of the present invention includes the main body 110, the handle 180, and the diffuser 200.

The main body 110 may include the gas outlet portion 150 for discharging the gas introduced from the outside, and the handle 180 may extend from the main body 110.

The diffuser 200 may be removably coupled to the main body 110, so that the gas discharged from the gas outlet portion 150 may flow into the diffuser 200 and the gas introduced into the diffuser 200 may be discharged to the outside.

The diffuser 200 may include the diffusing case 210 and the guide frame 240. In the diffusing case 210, the rear side 212 may be coupled to the main body 110, the gas discharged from the gas outlet portion 150 may be introduced into the diffusing case 210 through the gas inlet hole 215 defined in the rear side 212, the gas introduced into the diffusing case 210 may be discharged from the front side 211, and the inner diameter of the diffusing case 210 may increase toward the front side 211 from the rear side 212.

The guide frame 240 may be disposed inside the diffusing case 210 and may guide the flow of the gas introduced through the gas inlet hole 215. The guide frame 240 includes the diffusion portion 241, and the diffusion portion 241 is disposed to face the gas inlet hole 215 to diffuse the gas introduced through the gas inlet hole 215.

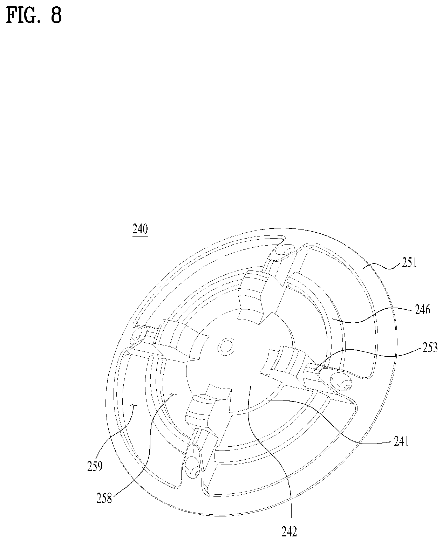

FIG. 8 shows the guide frame 240 according to an embodiment of the present invention. Referring to FIGS. 7 and 8, the guide frame 240 includes the diffusion portion 241 for diffusing the gas introduced from the gas inlet hole 215.

The diffuser 200 is disposed such that the inner diameter of the diffusing case 210 increases forwardly as shown in FIG. 7. Accordingly, the gas flowing from the rear side 212 of the diffusing case 210 to the front side 211 is discharged forward of the diffusing the case 210 in a state in which the flow cross-sectional area of the gas is increased to correspond to the inner diameter change of the diffusing case 210.

The increase of the flow cross-sectional area of the gas includes increase of a diameter of the flow cross-sectional area in addition to increase of the area itself. For example, referring to FIG. 7, the flow of gas introduced through the gas inlet hole 215 may have a flow cross-sectional area corresponding to a maximum diameter of the gas inlet hole 215.

On the other hand, the gas discharged from the front side 211 of the diffusing case 210 may have a flow cross-sectional area having a diameter corresponding to the maximum front surface, that is, the open surface of the diffusing case 210 by the shape of the diffusing case 210 whose inner diameter increases forwardly and by the diffusion portion 241 of the guide frame 240.

In addition, as shown in FIGS. 7 and 8, the diffusion portion 241 is disposed to face the gas inlet hole 215. Therefore, the gas introduced through the gas inlet hole 215 may be diffused such that the diameter of the flow cross-sectional area is increased by the diffusion portion 241.

Accordingly, the gas may be effectively diffused inside the diffusing case 210 through the guide frame 240 and may be provided to the user with a larger area, thereby improving the ease of use and efficiency.

In one example, referring to FIG. 8, the diffusion protrusion 242 protruding toward the gas inlet hole 215 may be disposed on a rear surface of the diffusion portion 241, and a protruding height of the diffusion protrusion 242 increases toward a center of the diffusion protrusion 242.

The diffusion protrusion 242 may rearwardly protrude from the rear surface of the diffusion portion 241, and the protruding height of the diffusion protrusion 242 may increase toward the center of the diffusion protrusion 242. That is, the diffusion protrusion 242 may have an approximately conical shape.

However, although a cross-section of the diffusion protrusion 242 is shown in FIG. 8 as a circular shape, the present disclosure is not limited thereto. The diffusion protrusion 242 may have a circular and polygonal cross-section as required.

An increase rate of the protruding height of the diffusion protrusion 242 that is increased from a circumference side to a central side of the diffusion protrusion 242 may be variously determined. For example, the diffusion protrusion 242 may increase in the protrusion height uniformly toward the central side from the circumference side thereof, may form a smooth curved surface as the increase rate gradually decreases, or may form a steep curved surface as the increase rate gradually increases. A specific shape of such diffusion protrusion 242 may be variously determined in terms of design.

The diffusion protrusion 242 may be manufactured separately from the diffusion portion 241 and coupled to a rear surface of the diffusion portion 241. Alternatively, diffusion protrusion 242 may be integrally formed with the diffusion portion 241, so that the diffusion protrusion 242 may correspond to the rear surface of the diffusion portion 241.

FIG. 8 shows a shape in which the diffusion protrusion 242 is integrally molded on the rear surface of the diffusion portion 241.

A diffusion degree and the flow of the gas may be improved by the diffusion protrusion 242. A level of diffusion in the radial direction of the diffusing case 210 of the gas introduced through the gas inlet hole 215 may be increased by the diffusion protrusion 242.

In addition, when the gas introduced through the gas inlet hole 215 directly collides with the rear surface of the diffusion portion 241 in a flat shape, the gas is diffused in the radial direction and collides with the rear surface of the diffusion portion 241, which result in turbulence or vortex.

The turbulence or vortex as described above may act as an element that inhibits the flow of the gas inside the diffusing case 210, and a noise of the diffuser 200 may be increased or a gas with low uniformity may be provided to the user, which may be disadvantageous.

Accordingly, the diffusion protrusion 242 is disposed on the rear surface of the diffusion portion 241, so that the gas introduced through the gas inlet hole 215 may be suppressed from flowing straight and colliding with the diffusion portion 241 and the diffusion of the gas in the radial direction may be naturally induced.

According to the invention, as shown in FIG. 8, the guide frame 240 has a ring shape, and a plurality of gas flow paths in which the diffusion portion 241 is located at a center are defined. The plurality of gas flow paths have different diameters and the gas flowing in the plurality of gas flow paths are separated from each other.

FIG. 8 illustrates a state in which the first flow path 258 and the second flow path 259 are defined in the guide frame 240. The first flow path 258 and the second flow path 259 may respectively have ring shapes, open in the front and rear direction, and may have different diameters.

The first flow path 258 and the second flow path 259 have the different diameters, but are able to form a mutual inclusion relationship as the diffusion portion 241 is disposed at a center of the guide frame 240.

For example, as shown in FIG. 8, the first flow path 258 may be defined inward of the second flow path 259. That is, the first flow path 258 and the second flow path 259 are defined to be concentric with each other, but have the different diameters, so that one may be defined to surround the other in a radially outward direction.

The number of flow paths defined by the guide frame 240 may be variously designed as needed. FIG. 8 shows the guide frame 240 having the first flow path 258 and the second flow path 259, but the present disclosure is not limited thereto. A third flow path, a fourth flow path, and the like may be additionally defined.

The first flow path 258 and the second flow path 259 may respectively correspond to paths through which the gas flows, and the gas flows in the first and second flow paths 258 and 259 may be separated from each other. As the diffusion portion 241 is centrally located and the plurality of gas flow paths that are substantially concentric and have the diameters different from each other are defined, the separate gas flows are formed respectively in the plurality of gas flow paths and the gas diffusion are effectively achieved.

For example, when the plurality of gas flow paths are not defined in the guide frame 240, the gas diffused through the diffusion portion 241 may flow along a single gas flow path that may be defined radially outward of the guide frame 240 or inside the guide frame 240. As described above, the gas flowing along the single gas flow path may be limited in an increase in a flow cross-sectional area thereof.

In addition, even though the gas flowing through one gas flow path as described above is discharged through the open surface of the diffusing case 210, the gas may have a gas flow of a distribution corresponding to a shape of one gas flow path. This means that the gas is provided to the user in a limited flow cross-sectional area regardless of an area of the open surface of the diffusing case 210, which may be disadvantageous in terms of the ease of use.

However, as the plurality of gas flow paths that are substantially concentric and have the different diameters are defined by the guide frame 240, the gas introduced through the gas inlet hole 215 may be diffused by the diffusion portion 241 and introduced and flowed into the plurality of gas flow paths.

Accordingly, the flow cross-sectional area of the gas inside the diffusing case 210 may be effectively increased, and a discharge cross-sectional area of the gas, and the diffusion degree and the uniformity of the gas on the basis of the open surface of the diffusing case 210 may be effectively improved, thereby effectively improving the ease of use.

In one example, at least one of the plurality of gas flow paths may have a diameter larger than the diameter of the gas inlet hole 215. FIG. 7 shows that the second flow path 259 of the guide frame 240 has a larger diameter than the gas inlet hole 215.

For example, the second flow path 259 having a diameter larger than that of the first flow path 258 and disposed outward of the first flow path 258 may have an inner diameter or an outer diameter larger than the diameter of the gas inlet hole 215.

Even when the gas introduced through the gas inlet hole 215 is diffused in the radial direction by the diffusion portion 241, when the gas flow path through which the gas flows has a diameter equal to or less than the gas inlet hole 215, the flow cross-sectional area of the gas discharged from the diffuser 200 through the gas flow path eventually becomes not differ significantly from the flow cross-sectional area of the gas introduced into the gas inlet hole 215.

That is, the inner diameter of the diffusing case 210 increases forwardly, the gas introduced from the gas inlet hole 215 diffuses in the radial direction by the diffusion portion 241, and at least some of the gas flow paths defined by the guide frame 240 have diameters larger than that of the gas inlet hole 215. Thus, as the gas introduced into the diffuser 200 flows inside the diffuser 200, the flow cross-sectional area of the gas may be effectively expanded and the gas may be provided to the user with a larger area, thereby improving the ease of use.

FIG. 8, the plurality of gas flow paths include the first flow path 258 and the second flow path 259, and the guide frame 240 may further include the first guide 246 and the second guide 251.

The first guide 246 may be formed in the ring shape, the diffusion portion 241 may be located at a center of the first guide 246, and the first flow path 258 may be defined between the first guide 246 and the diffusion portion 241. The second guide 251 may be formed in the ring shape, the diffusion portion 241 and the first guide 246 may be located at a center of the second guide 251, and the second flow path 259 may be defined between the second guide 251 and the first guide 246.

The gas introduced through the gas inlet hole 215 may flow to the front side 211 of the diffusing case 210 through the first flow path 258 and the second flow path 259.

The first guide 246 and the second guide 251 may be formed in the ring shapes and may have the diffusion portion 241 located centers thereof. That is, the diffusion portion 241, the first guide 246, and the second guide 251 may be concentric and may be spaced apart from each other.

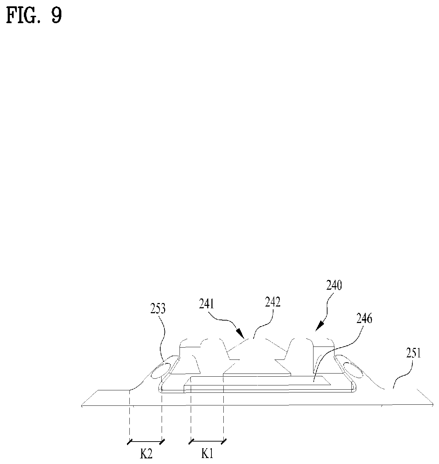

FIG. 9 shows the first guide 246 having a separation distance K1 from the diffusion portion 241 and the second guide 251 having a separation distance K2 from the first guide 246.

The first flow path 258 and the second flow path 259 may be defined to have the ring shapes and spaced apart from each other by the shapes and arrangement of the first guide 246 and the second guide 251. Therefore, a width of the first flow path 258 may correspond to the separation distance K1 between the diffusion portion 241 and the first guide 246, and a width of the second flow path 259 may correspond to the separation distance K2 between the first guide 246 and the second guide 251.

The first flow path 258 may be defined between the diffusion portion 241 and the first guide 246 to surround the diffusion portion 241, and the second flow path 259 may be defined between the first guide 246 and the second guide 251 to outwardly surround the first flow path 258.

The guide frame 240 includes the first guide 246 and the second guide 251 in addition to the diffusion portion 241. The first flow path 258 and the second flow path 259 may be effectively defined through the shapes and the arrangement relationship of the first guide 246 and the second guide 251.

In addition, as the first flow path 258 and the second flow path 259 are formed in the ring shapes and the diameters thereof are gradually increased in a stepwise manner, the gas that is uniformly dispersed along the circumferential direction and also uniformly dispersed in the radial direction of the diffusing case 210 from the open surface of the diffusing case 210 may be provide to the user with the large area.

In one example, the second flow path 259 has an outer diameter larger than the diameter of the gas inlet hole 215, so that the gas introduced through the gas inlet hole 215 may be diffused and flowed.

As described above, at least one of the plurality of gas flow paths have the diameter larger than that of the gas inlet hole 215, thereby contributing to the gas flow diffusion.

As above, the guide frame 240 may have the first flow path 258 and the second flow path 259. The second flow path 259 may be defined to surround the first flow path 258 while having a diameter larger than that of the first flow path 258, and may have an outer diameter larger than the diameter of the gas inlet hole 215. That is, the inner diameter of the second guide 251 may be larger than the diameter of the gas inlet hole 215.

Therefore, the gas introduced through the second flow path 259 may have a flow cross-sectional area having a diameter at least larger than that of the gas inlet hole 215, and the gas having a wide cross-sectional area may be effectively provided to the user.

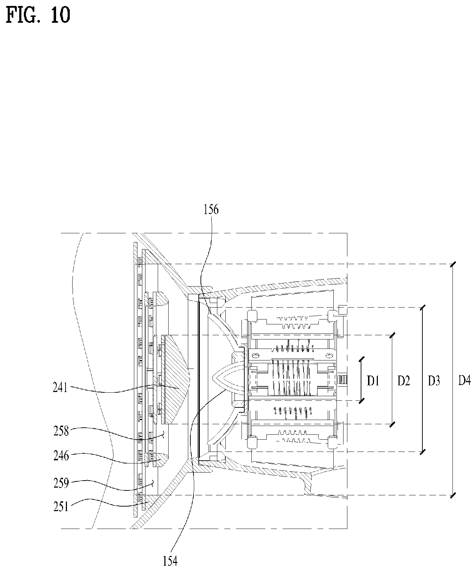

In one example, FIG. 9 shows a view of the guide frame 240 according to an embodiment of the present invention viewed from the side, and FIG. 10 shows the interior of the diffuser 200 coupled to the main body 110.

Referring to FIGS. 9 and 10, the first guide 246 is forwardly spaced apart from the diffusion portion 241, and the second guide 251 is forwardly spaced apart from the first guide 246.

That is, the guide frame 240 may be in a form protruding forward in a stepwise manner in an outer radial direction from the center thereof. Accordingly, a space through which the gas introduced through the gas inlet hole 215 may be diffused by the diffusion portion 241 and introduced into the first flow path 258 and the second flow path 259 may be secured.

Specifically, when the diffusion portion 241, the first guide 246 and the second guide 251 are flush with each other, that is, when the diffusion portion 241, the first guide 246, and the second guide 251 are arranged at the same position in the front and rear direction, a separation distance between the second flow path 259 and the inner surface of the diffusing case 210 may be smaller than a separation distance between the first flow path 258 and the inner surface of the diffusing case 210.

That is, a space defined between the second flow path 259 and the inner surface of the diffusing case 210 and defined rearward of the second flow path 259 may have a relatively small area and may interrupt the gas diffused by the diffusion portion 241 from being sufficiently introduced into the second flow path 259.

In this case, the gas introduced through the gas inlet hole 215 may be concentrated into the first flow path 258. Consequently, the gas discharged from the front side 211 of the diffusing case 210 may be more concentrated in a region corresponding to the first flow path 258 and provided to the user. This may be an adverse result for the gas diffusion effect of the diffuser 200.

Therefore, an inlet of the second flow path 259, which is located farther from the center of the gas inlet hole 215 than the first flow path 258, is defined to be forwardly spaced apart from the first flow path 258, so that the gas introduced from the gas inlet hole 215 may be sufficiently introduced into the second flow path 259, thereby effectively improving the diffusion effect of the gas.

In one example, the diffuser may further include the light irradiator 260 disposed on the front surfaces of the diffusion portion 241, the first guide 246, and the second guide 251 to irradiate the light toward the front side 211 of the diffusing case 210.

The guide frame 240 includes the diffusion portion 241, the first guide 246, and the second guide 251, which are sequentially and forwardly spaced apart from each other in the direction away from the center of the guide frame 240. The light irradiator 260 disposed on the front surface of the guide frame 240 may have portions sequentially and forwardly spaced apart from each other in the direction away from the center of the light irradiator 260 like the shape of the guide frame 240.

Accordingly, the portions of the light irradiator 260 may have an arrangement structure corresponding to a sphere surface as the portions are located forwardly in the direction away from the center of the light irradiator 260, which may have similarity to the user's head.

That is, the light irradiator 260 is disposed on the front surface of the guide frame 240 and has the arrangement structure of the portions thereof substantially corresponding to the sphere surface, thereby enabling uniform light irradiation to the scalp and the hair of the user.

The guide frame 240 may further include the guide connector 253. The guide connector 253 may extend along the radial direction of the guide frame 240 to connect the diffusion portion 241, the first guide 246, and the second guide 251 with each other.

The guide connector 253 may be made of a material the same as or different from a material of the diffusion portion 241, the first guide 246, and the second guide 251. The guide connector 253 may be separately manufactured and have a coupling relationship with the diffusion portion 241, the first guide 246, and the second guide 251, or may be integrally molded with the diffusion portion 241 and the like.

That is, in the guide frame 240, the diffusion portion 241, the first guide 246, and the second guide 251 connected to each other through the guide connector 253 may be integrally manufactured and handled.

The shape, the arrangement structure, and the like of the guide frame 240 are determined as the guide frame 240 is constituted by the plurality of components. The guide frame 240 constituted by the plurality of components may increase a time required during a manufacturing process or an assembly process, which may be disadvantageous.

Accordingly, the present disclosure may improve the handling of the guide frame 240 in the manufacturing process of the guide frame 240 or the assembly process of the diffuser 200 by allowing the guide frame 240 to have the plurality of components separated from each other, such as the diffusion portion 241, the first guide 246 and the second guide 251 and allowing the plurality of components to be considered and handled as a single component through the guide connector 253.

The diffuser 200 may further include the discharge cover 300. The discharge cover 300 may be disposed at the front side 211 of the diffusing case 210 and may include the gas discharge hole 305 through which the gas introduced into the diffusing case 210 is discharged to the outside. In addition, the discharge cover 300 may include the massage protrusion 310 that protrudes forward to press the target located in front of the discharge cover 300.

As described above, the plurality of gas discharge holes 305 may be defined in the discharge cover 300, and the plurality of gas discharge holes 305 may be arranged to be distributed throughout the front surface of the discharge cover 300. Accordingly, the gas flowing through the first flow path 258 and the second flow path 259 may be dispersed throughout the discharge cover 300 and discharged. When the discharge cover 300 is included in the diffuser 200, the front surface of the diffusing case 210 described above, that is, the open surface may be understood as the same concept as the discharge cover 300.

In one example, the diffuser 200 may provide the massage effect by pressing the user's scalp through the plurality of massage protrusions 310 arranged on the discharge cover 300. That is, an embodiment of the present invention not only may provide the gas diffused over a large area through the diffuser 200, but also may provide the massage effect to the user's scalp and provide the care effect to the scalp and the hair of the user through the light irradiator 260 described above.

In one example, the main body 110 includes the gas outlet portion 150. An entirety of the gas outlet portion 150 may have a hole shape, or the gas outlet portion 150 may include the center portion 154 and the side portion 156 as shown in FIG. 10.

The center portion 154 may be defined at approximately the center of the gas outlet portion 150 and the gas may be discharged through the center portion 154. Further, the side portion 156 may have the ring shape and may surround the center portion 154, and the gas may be discharged through the side portion 156 along with the center hole.

The center portion 154 and the side portion 156 may be defined in the form of holes that open in the front and rear direction. The center portion 154 may correspond to a hole defined at the center of the discharge base 152 of the gas outlet portion 150, and the guide cone for improving the gas flow may be disposed at the center of the center portion 154. In this case, the center portion 154 may correspond to a hole having a ring-shaped cross-section.

The side portion 156 may be defined to be outwardly spaced apart from the center portion 154 in the radial direction of the gas outlet portion 150, and may have a cross-section in a shape of a ring surrounding the center portion 154. The side portion 156 may be understood as a hole defined between the discharge base 152 and the outer wall front end 112 of the main body 110.

The diffusion portion 241 may be disposed to face the center portion 154 and may have the diameter larger than that of the center portion 154.

When the center portion 154 and the side portion 156 are defined in the gas outlet portion 150, the diffusion portion 241 may be positioned to face the center portion 154 and may have the diameter larger than that of the center portion 154 to the diffuse gas discharged from the center portion 154 in the radial direction.

When the gas discharged from the center portion 154 is diffused in the radial direction by the diffusion portion 241, for example, the diffusion protrusion 242, the gas of the center portion 154 diffused by the diffusion portion 241 diffuses the gas discharged from the side portion 156 outward in the radial direction. FIG. 7 shows the flow of the gas diffused as described above.

The diffusion portion 241 diffuses at least the gas of the center portion 154 outward in the radial direction in consideration of the relationship between the center portion 154 and the side portion 156, so that not only the gas of the center portion 154, but also the gas of the side portion 156 may be effectively diffused outward in the radial direction.

In one example, in the diffusing case 210, the diameter of the gas inlet hole 215 is equal to or larger than the diameter of the side portion 156, so that the gas discharged from the center portion 154 and the side portion 156 may be introduced into the diffusing case 210. Further, the diffusion portion 241 may have the diameter larger than the diameter of the center portion 154 to diffuse the gas introduced from the center portion 154.

Specifically, the hair dryer 100 may be disposed such that the gas inlet hole 215 faces the gas outlet portion 150, and the diameter of the gas inlet hole 215 may be equal to or larger than that of the gas outlet portion 150. Accordingly, not only the gas of the center portion 154 discharged from the gas outlet portion 150, but also the gas of the side portion 156 may also be introduced into the gas inlet hole 215 with minimal leakage to the outside.

Further, as the above-described coupling sleeve 224 is disposed to extend rearward from the circumference of the gas inlet hole 215 to surround the front end 112 of the main body 110, an entirety of the gas discharged from the gas outlet portion 150 may be introduced into the gas inlet hole 215.

In the above structure, as the diffusion portion 241 has the diameter larger than that of the center portion 154, not only the gas of the center portion 154, but also the gas of the side portion 156 may be effectively diffused by being continuously affected by the gas diffusion of the center portion 154. FIG. 10 shows the diffusion portion 241 having a diameter D2 larger than a diameter D1 of the center portion 154.

In one example, the diffusion portion 241 may have the diameter smaller than the inner diameter of the side portion 156, and the side portion 156 may face the first flow path 258 in the forward direction.

FIG. 10 shows a state in which, as the diameter D2 of the diffusion portion 241 is smaller than a diameter D3 of the side portion 156, the side portion 156 faces at least a portion of the first flow path 258 in the front and rear direction.

When the diameter D2 of the diffusion portion 241 is larger than the inner diameter D3 of the side portion 156, the gas discharged from the side portion 156 is not able to flow straight and is diffused and flows in the radial direction by the diffusion portion 241 and a region where the straight flow is restricted by the diffusion portion 241 becomes too large, so that an amount of gas on a central side on the cross-section of the gas discharged forwardly of the diffuser 200 may be reduced.

In this case, the gas amount is insufficient on the central side of the gas discharged from the diffuser 200, which may lower a sense of use. Further, a portion of the flow of the gas discharged from the diffuser 200 may be induced to the central side to reduce the flow cross-sectional area of the gas, which may be disadvantageous.

Therefore, the gas discharged from the center portion 154 of the gas outlet portion 150 may be restricted from flowing straight by the diffusion portion 241 and may be diffused in the radial direction. Further, the side portion 156 is disposed so as not to overlap with the diffusion portion 241, so that a portion of the gas may flow straight into the first flow path 258, and the remaining portion of the gas may be discharged from the center portion 154 and diffused along the radial direction under an influence of the gas diffused by the diffusion portion 241. The diameter D1 of the diffusion portion 241, the diameter D2 of the center portion 154, and the diameter D3 of the side portion 156 as described above may be variously determined in terms of design.

In one example, referring to FIG. 10, the second flow path 259 may have the inner diameter larger than the outer diameter of the side portion 156, so that the gas introduced through the center portion 154 and the side portion 156 may be diffused and flowed.

FIG. 10 shows that an inner diameter D4 of the second flow path 259 is larger than the outer diameter D3 of the side portion 156.

An embodiment of the present invention allows the portion of the gas discharged from the side portion 156 to flow straight into the first flow path 258. However, the outer diameter of the second flow path 259, that is, the inner diameter D4 of the second guide 251 is at least larger than the outer diameter D3 of the side portion 156, so that the diffusion may be induced such that an entirety of the gas discharged from the gas outlet portion 150, including the gas discharged from the side portion 156, has a larger flow cross-sectional area.

FIG. 10 shows the guide frame 240 in which the second guide 251 has the inner diameter D3 larger than the outer diameter D3 of the side portion 156 to induce the diffusion of the entirety of the gas discharged from the gas outlet portion 150, and in which the second guide 251 is located forwardly of the first guide 246 to secure a space for the gas to flow into the second flow path 259 from the inner surface of the diffusing case 210.

Although a specific embodiment of the present invention has been illustrated and described above, those of ordinary skill in the art to which the present invention pertains will appreciate that various modifications are possible within the limits without departing from the scope of the following claims.

## Claims

1. A diffuser (200) comprising:
a diffusing case (210) having a rear side removably coupled to a main body of a hair dryer, wherein gas discharged from the main body is introduced into the diffusing case (210) through a gas inlet hole (215) located at the rear side, wherein the gas introduced into the diffusing case (210) is discharged from a front side of the diffusing case (210), and wherein an inner diameter of the diffusing case (210) increases forwardly; and
a guide frame (240) disposed inside the diffusing case (210) to guide flow of the gas introduced through the gas inlet hole,
wherein the guide frame (240) includes a diffusion portion (241) disposed to face the gas inlet hole (215) to diffuse the gas introduced through the gas inlet hole (215),
the guide frame (240) includes a plurality of gas flow paths (258, 259) having a ring shape, wherein the diffusion portion (241) is located at a center of the plurality of gas flow paths, and
the plurality of gas flow paths (258, 259) have different diameters to each other to divide gas flows from each other, and
wherein the diffusion portion (241) has a diffusion protrusion (242) protruding toward the gas inlet hole (215) on a rear surface of the diffusion portion (241) facing the gas inlet hole (215),
**characterized in that** a protrusion height of the diffusion protrusion (242) increases toward a center of the diffusion protrusion (242).

2. The diffuser of claim 1, wherein at least one of the plurality of gas flow paths has a diameter larger than a diameter of the gas inlet hole (215).

3. The diffuser of claim 1, wherein the plurality of gas flow paths (258, 259) include a first flow path (258) and a second flow path (259),
wherein the guide frame (240) further includes:
a first guide (246) formed in a ring shape to have the diffusion portion (241) located at a center of the first guide, wherein the first flow path (258) is defined between the first guide (246) and the diffusion portion (241); and
a second guide (251) formed in a ring shape to have the diffusion portion (241) and the first guide (246) at a center of the second guide (251), wherein the second flow path (259) is defined between the first guide (246) and the second guide (251),
wherein the gas introduced through the gas inlet hole (215) flows to the front side of the diffusing case (210) through the first flow path (258) and the second flow path (259).

4. The diffuser of claim 3, wherein the second flow path (259) has an outer diameter larger than a diameter of the gas inlet hole (215) to diffuse the gas introduced through the gas inlet hole (215).

5. The diffuser of claim 3, wherein the first guide (246) is located forwardly of the diffusion portion (241), and the second guide (251) is located forwardly of the first guide (246).

6. The diffuser of claim 5, further comprising:
a light irradiator disposed on each front surface of the diffusion portion (241), the first guide (246), and the second guide (251) to irradiate light toward the front side of the diffusing case (210).

7. The diffuser of claim 3, wherein the guide frame (240) further includes:
a guide connector extending along a radial direction of the guide frame (240) to connect the diffusion portion (241), the first guide (246), and the second guide (251) to each other.

8. The diffuser of claim 1, further comprising:
a discharge cover disposed at the front side of the diffusing case (210), wherein the discharge cover includes a gas discharge hole for discharging the gas introduced into the diffusing case (210) to outside,
wherein the discharge cover includes a massage protrusion protruding forward.

9. A hair dryer comprising:
a main body (110) including a gas outlet portion (150) for discharging gas therethrough;
a handle (180) extending from the main body (110); and
a diffuser (200) removably coupled to the main body (110) to introduce the gas discharged from the gas outlet portion (150) therein and discharge the gas introduced therein to outside,
wherein the diffuser (200) includes:
a diffusing case (210) having a rear side removably coupled to the main body, wherein the gas discharged from the gas outlet portion (150) is introduced into the diffusing case (210) through a gas inlet hole (215) located at the rear side, wherein the gas introduced into the diffusing case (210) is discharged from a front side of the diffusing case (210), and wherein an inner diameter of the diffusing case (210) increases forwardly; and
a guide frame (240) disposed inside the diffusing case (210) to guide flow of the gas introduced through the gas inlet hole (215),
wherein the guide frame (240) includes a diffusion portion (241) disposed to face the gas inlet hole (215) to diffuse the gas introduced through the gas inlet hole (215),
the guide frame (240) includes a plurality of gas flow paths (258, 259) having a ring shape, wherein the diffusion portion (241) is located at a center of the plurality of gas flow paths, and
the plurality of gas flow paths (258, 259) have different diameters to each other to divide gas flows from each other, and
wherein the diffusion portion (241) has a diffusion protrusion (242) protruding toward the gas inlet hole (215) on a rear surface of the diffusion portion (241) facing the gas inlet hole (215),
**characterized in that** a protrusion height of the diffusion protrusion (242) increases toward a center of the diffusion protrusion (242).

10. The hair dryer of claim 9, wherein the gas outlet portion (150) includes:
a center portion (154) for discharging the gas therethrough; and
a side portion (156) having a ring shape and surrounding the center portion (154), wherein the gas is discharged through the side portion (156) and the center portion (154),
wherein the diffusion portion (241) is disposed to face the center portion and has a diameter larger than a diameter of the center portion (156) to diffuse the gas introduced from the center portion (154).

11. The hair dryer of claim 10, wherein a diameter of the gas inlet hole (215) is equal to or larger than a diameter of the side portion (156), so that the gas discharged from the center portion (154) and the side portion (156) flows into the diffusing case (210).

12. The hair dryer of claim 11, wherein the guide frame (240) further includes:
a first guide (246) formed in a ring shape to have the diffusion portion (241) located at a center of the first guide (246), wherein a first flow path (258) for flowing the gas therethrough is defined between the first guide (246) and the diffusion portion (241); and
a second guide (251) formed in a ring shape to have the diffusion portion (241) and the first guide (246) at a center of the second guide (251), wherein a second flow path (259) for flowing the gas therethrough is defined between the second guide (251) and the diffusion portion (241),
wherein the diffusion portion (241) has a diameter smaller than an inner diameter of the side portion (156), and
wherein the side portion (156) is positioned to face at least a portion of the first flow path (258) in a forward direction.

13. The hair dryer of claim 12, wherein the second flow path (259) has an inner diameter larger than an outer diameter of the side portion (156), so that the gas introduced through the center portion (154) and the side portion (156) is diffused and flows.

## Patentansprüche

1. Diffusor (200), umfassend:
ein Diffusorgehäuse (210) mit einer Rückseite, die lösbar mit einem Hauptkörper eines Haartrockners gekoppelt ist, wobei aus dem Hauptkörper abgegebenes Gas in das Diffusorgehäuse (210) durch ein an der Rückseite angeordnetes Gaseinlassloch (215) eingeleitet wird, wobei das in das Diffusorgehäuse (210) eingeleitete Gas von einer Vorderseite des Diffusorgehäuses (210) abgegeben wird, und wobei ein Innendurchmesser des Diffusorgehäuses (210) nach vorn zunimmt; und
einen Führungsrahmen (240), der innerhalb des Diffusorgehäuses (210) angeordnet ist, um die Strömung des durch das Gaseinlassloch eingeleiteten Gases zu führen,
wobei der Führungsrahmen (240) einen Diffusionsabschnitt (241) umfasst, der so angeordnet ist, dass er dem Gaseinlassloch (215) gegenüberliegt, um das durch das Gaseinlassloch (215) eingeleitete Gas zu diffundieren,
der Führungsrahmen (240) eine Vielzahl von gasförmigen Strömungspfaden (258, 259) in Ringform umfasst, wobei der Diffusionsabschnitt (241) in einem Zentrum der Vielzahl von gasförmigen Strömungspfaden angeordnet ist, und
die Vielzahl von gasförmigen Strömungspfaden (258, 259) unterschiedliche Durchmesser zueinander aufweisen, um Gasströmungen voneinander zu trennen, und
wobei der Diffusionsabschnitt (241) einen Diffusionsvorsprung (242) aufweist, der an einer dem Gaseinlassloch (215) zugewandten Rückfläche des Diffusionsabschnitts (241) in Richtung des Gaseinlasslochs (215) vorsteht,
**dadurch gekennzeichnet, dass** eine Vorsprunghöhe des Diffusionsvorsprungs (242) zu einem Zentrum des Diffusionsvorsprungs (242) hin zunimmt.

2. Diffusor nach Anspruch 1, wobei zumindest einer der Vielzahl von gasförmigen Strömungspfaden einen Durchmesser aufweist, der größer ist als ein Durchmesser des Gaseinlasslochs (215).

3. Diffusor nach Anspruch 1, wobei die Vielzahl von gasförmigen Strömungspfaden (258, 259) einen ersten Strömungspfad (258) und einen zweiten Strömungspfad (259) umfasst,
wobei der Führungsrahmen (240) ferner umfasst:
eine erste Führung (246), die in Ringform ausgebildet ist, sodass der Diffusionsabschnitt (241) im Zentrum der ersten Führung angeordnet ist, wobei der erste Strömungspfad (258) zwischen der ersten Führung (246) und dem Diffusionsabschnitt (241) definiert ist; und
eine zweite Führung (251), die in Ringform ausgebildet ist, sodass der Diffusionsabschnitt (241) und die erste Führung (246) im Zentrum der zweiten Führung (251) angeordnet sind, wobei der zweite Strömungspfad (259) zwischen der ersten Führung (246) und der zweiten Führung (251) definiert ist,
wobei das durch das Gaseinlassloch (215) eingeleitete Gas zur Vorderseite des Diffusorgehäuses (210) durch den ersten Strömungspfad (258) und den zweiten Strömungspfad (259) strömt.

4. Diffusor nach Anspruch 3, wobei der zweite Strömungspfad (259) einen Außendurchmesser aufweist, der größer ist als ein Durchmesser des Gaseinlasslochs (215), um das durch das Gaseinlassloch (215) eingeleitete Gas zu diffundieren.

5. Diffusor nach Anspruch 3, wobei die erste Führung (246) vor dem Diffusionsabschnitt (241) angeordnet ist und die zweite Führung (251) vor der ersten Führung (246) angeordnet ist.

6. Diffusor nach Anspruch 5, ferner umfassend:
einen Lichtstrahler, der auf jeder Vorderfläche des Diffusionsabschnitts (241), der ersten Führung (246) und der zweiten Führung (251) angeordnet ist, um Licht zur Vorderseite des Diffusorgehäuses (210) hin abzustrahlen.

7. Diffusor nach Anspruch 3, wobei der Führungsrahmen (240) ferner umfasst:
einen Führungsverbinder, der sich entlang einer radialen Richtung des Führungsrahmens (240) erstreckt, um den Diffusionsabschnitt (241), die erste Führung (246) und die zweite Führung (251) miteinander zu verbinden.

8. Diffusor nach Anspruch 1, ferner umfassend:
eine Abgabedeckung, die an der Vorderseite des Diffusorgehäuses (210) angeordnet ist, wobei die Abgabedeckung ein Gasabgabeloch umfasst, um das in das Diffusorgehäuse (210) eingeleitete Gas nach außen abzugeben,
wobei die Abgabedeckung einen Massagevorsprung umfasst, der nach vorn vorsteht.

9. Haartrockner, umfassend:
einen Hauptkörper (110), der einen Gasauslassabschnitt (150) umfasst, um Gas dadurch abzugeben; einen Griff (180), der sich vom Hauptkörper (110) erstreckt; und
einen Diffusor (200), der lösbar mit dem Hauptkörper (110) gekoppelt ist, um das vom Gasauslassabschnitt (150) abgegebene Gas in ihn einzuleiten und das in ihn eingeleitete Gas nach außen abzugeben,
wobei der Diffusor (200) umfasst:
ein Diffusorgehäuse (210) mit einer Rückseite, die lösbar mit dem Hauptkörper gekoppelt ist, wobei das vom Gasauslassabschnitt (150) abgegebene Gas in das Diffusorgehäuse (210) durch ein an der Rückseite angeordnetes Gaseinlassloch (215) eingeleitet wird, wobei das in das Diffusorgehäuse (210) eingeleitete Gas von einer Vorderseite des Diffusorgehäuses (210) abgegeben wird, und wobei ein Innendurchmesser des Diffusorgehäuses (210) nach vorn zunimmt; und
einen Führungsrahmen (240), der innerhalb des Diffusorgehäuses (210) angeordnet ist, um die Strömung des durch das Gaseinlassloch (215) eingeleiteten Gases zu führen,
wobei der Führungsrahmen (240) einen Diffusionsabschnitt (241) umfasst, der so angeordnet ist, dass er dem Gaseinlassloch (215) gegenüberliegt, um das durch das Gaseinlassloch (215) eingeleitete Gas zu diffundieren,
der Führungsrahmen (240) eine Vielzahl von gasförmigen Strömungspfaden (258, 259) in Ringform umfasst, wobei der Diffusionsabschnitt (241) in einem Zentrum der Vielzahl von gasförmigen Strömungspfaden angeordnet ist,
und
die Vielzahl von gasförmigen Strömungspfaden (258, 259) unterschiedliche Durchmesser zueinander aufweisen, um Gasströmungen voneinander zu trennen, und
wobei der Diffusionsabschnitt (241) einen Diffusionsvorsprung (242) aufweist, der an einer dem Gaseinlassloch (215) zugewandten Rückfläche des Diffusionsabschnitts (241) in Richtung des Gaseinlasslochs (215) vorsteht,
**dadurch gekennzeichnet, dass** eine Vorsprunghöhe des Diffusionsvorsprungs (242) zu einem Zentrum des Diffusionsvorsprungs (242) hin zunimmt.

10. Haartrockner nach Anspruch 9, wobei der Gasauslassabschnitt (150) umfasst:
einen Mittelabschnitt (154), um das Gas dadurch abzugeben; und
einen Seitenabschnitt (156) in Ringform, der den Mittelabschnitt (154) umgibt, wobei das Gas durch den Seitenabschnitt (156) und den Mittelabschnitt (154) abgegeben wird,
wobei der Diffusionsabschnitt (241) so angeordnet ist, dass er dem Mittelabschnitt gegenüberliegt, und einen Durchmesser aufweist, der größer ist als ein Durchmesser des Mittelabschnitts (156), um das vom Mittelabschnitt (154) eingeleitete Gas zu diffundieren.

11. Haartrockner nach Anspruch 10, wobei ein Durchmesser des Gaseinlasslochs (215) gleich oder größer ist als ein Durchmesser des Seitenabschnitts (156), sodass das vom Mittelabschnitt (154) und dem Seitenabschnitt (156) abgegebene Gas in das Diffusorgehäuse (210) strömt.

12. Haartrockner nach Anspruch 11, wobei der Führungsrahmen (240) ferner umfasst:
eine erste Führung (246), die in Ringform ausgebildet ist, sodass der Diffusionsabschnitt (241) im Zentrum der ersten Führung (246) angeordnet ist, wobei ein erster Strömungspfad (258) zum Durchströmen des Gases zwischen der ersten Führung (246) und dem Diffusionsabschnitt (241) definiert ist; und
eine zweite Führung (251), die in Ringform ausgebildet ist, sodass der Diffusionsabschnitt (241) und die erste Führung (246) im Zentrum der zweiten Führung (251) angeordnet sind, wobei ein zweiter Strömungspfad (259) zum Durchströmen des Gases zwischen der zweiten Führung (251) und dem Diffusionsabschnitt (241) definiert ist,
wobei der Diffusionsabschnitt (241) einen Durchmesser aufweist, der kleiner ist als ein Innendurchmesser des Seitenabschnitts (156), und
wobei der Seitenabschnitt (156) so positioniert ist, dass er in einer Vorwärtsrichtung zumindest einem Teil des ersten Strömungspfads (258) gegenüberliegt.

13. Haartrockner nach Anspruch 12, wobei der zweite Strömungspfad (259) einen Innendurchmesser aufweist, der größer ist als ein Außendurchmesser des Seitenabschnitts (156), sodass das durch den Mittelabschnitt (154) und den Seitenabschnitt (156) eingeleitete Gas diffundiert wird und strömt.

## Revendications

1. Diffuseur (200), comprenant :
un boîtier de diffuseur (210) ayant une face arrière couplée de manière détachable à un corps principal d'un sèche-cheveux, un gaz émis depuis le corps principal étant introduit dans le boîtier de diffuseur (210) par un orifice d'entrée de gaz (215) disposé sur la face arrière, le gaz introduit dans le boîtier de diffuseur (210) étant émis depuis une face avant du boîtier de diffuseur (210), et un diamètre intérieur du boîtier de diffuseur (210) augmentant vers l'avant ; et
un châssis de guidage (240) disposé à l'intérieur du boîtier de diffuseur (210) pour guider l'écoulement du gaz introduit par l'orifice d'entrée de gaz,
le châssis de guidage (240) comprenant une section de diffusion (241) disposée de manière à faire face à l'orifice d'entrée de gaz (215) afin de diffuser le gaz introduit par l'orifice d'entrée de gaz (215),
le châssis de guidage (240) comprenant une pluralité de chemins d'écoulement gazeux (258, 259) en forme annulaire, la section de diffusion (241) étant disposée au centre de la pluralité de chemins d'écoulement gazeux, et
la pluralité de chemins d'écoulement gazeux (258, 259) ayant des diamètres différents les uns des autres afin de séparer des écoulements de gaz les uns des autres, et
la section de diffusion (241) ayant une saillie de diffusion (242) faisant saillie, sur une face arrière de la section de diffusion (241) faisant face à l'orifice d'entrée de gaz (215), en direction de l'orifice d'entrée de gaz (215),
**caractérisé en ce qu'**une hauteur de saillie de la saillie de diffusion (242) augmente vers un centre de la saillie de diffusion (242).

2. Diffuseur selon la revendication 1, dans lequel au moins un des chemins d'écoulement gazeux de la pluralité a un diamètre supérieur à un diamètre de l'orifice d'entrée de gaz (215).

3. Diffuseur selon la revendication 1, dans lequel la pluralité de chemins d'écoulement gazeux (258, 259) comprend un premier chemin d'écoulement (258) et un second chemin d'écoulement (259),
le châssis de guidage (240) comprenant en outre :
un premier guide (246) formé en forme annulaire de sorte que la section de diffusion (241) soit disposée au centre du premier guide, le premier chemin d'écoulement (258) étant défini entre le premier guide (246) et la section de diffusion (241) ; et
un second guide (251) formé en forme annulaire de sorte que la section de diffusion (241) et le premier guide (246) soient disposés au centre du second guide (251), le second chemin d'écoulement (259) étant défini entre le premier guide (246) et le second guide (251),
le gaz introduit par l'orifice d'entrée de gaz (215) s'écoulant vers la face avant du boîtier de diffuseur (210) à travers le premier chemin d'écoulement (258) et le second chemin d'écoulement (259).

4. Diffuseur selon la revendication 3, dans lequel le second chemin d'écoulement (259) a un diamètre extérieur supérieur à un diamètre de l'orifice d'entrée de gaz (215) afin de diffuser le gaz introduit par l'orifice d'entrée de gaz (215).

5. Diffuseur selon la revendication 3, dans lequel le premier guide (246) est disposé en avant de la section de diffusion (241) et le second guide (251) est disposé en avant du premier guide (246).

6. Diffuseur selon la revendication 5, comprenant en outre :
un dispositif d'émission lumineuse disposé sur chaque face avant de la section de diffusion (241), du premier guide (246) et du second guide (251), afin d'émettre une lumière vers la face avant du boîtier de diffuseur (210).

7. Diffuseur selon la revendication 3, dans lequel le châssis de guidage (240) comprend en outre :
un connecteur de guidage s'étendant le long d'une direction radiale du châssis de guidage (240) afin de relier entre eux la section de diffusion (241), le premier guide (246) et le second guide (251).

8. Diffuseur selon la revendication 1, comprenant en outre :
un couvercle de sortie disposé à l'avant du boîtier de diffuseur (210), le couvercle de sortie comprenant un orifice de sortie de gaz afin d'émettre vers l'extérieur le gaz introduit dans le boîtier de diffuseur (210),
le couvercle de sortie comprenant une saillie de massage faisant saillie vers l'avant.

9. Un sèche-cheveux, comprenant :
un corps principal (110) comprenant une section de sortie de gaz (150) afin d'émettre du gaz par celle-ci ; une poignée (180) s'étendant depuis le corps principal (110) ; et
un diffuseur (200) couplé de manière détachable au corps principal (110) afin d'y introduire le gaz émis par la section de sortie de gaz (150) et d'émettre vers l'extérieur le gaz introduit dans celui-ci,
le diffuseur (200) comprenant :
un boîtier de diffuseur (210) ayant une face arrière couplée de manière détachable au corps principal, le gaz émis par la section de sortie de gaz (150) étant introduit dans le boîtier de diffuseur (210) par un orifice d'entrée de gaz (215) disposé sur la face arrière, le gaz introduit dans le boîtier de diffuseur (210) étant émis depuis une face avant du boîtier de diffuseur (210), et un diamètre intérieur du boîtier de diffuseur (210) augmentant vers l'avant ; et
un châssis de guidage (240) disposé à l'intérieur du boîtier de diffuseur (210) pour guider l'écoulement du gaz introduit par l'orifice d'entrée de gaz (215),
le châssis de guidage (240) comprenant une section de diffusion (241) disposée de manière à faire face à l'orifice d'entrée de gaz (215) afin de diffuser le gaz introduit par l'orifice d'entrée de gaz (215),
le châssis de guidage (240) comprenant une pluralité de chemins d'écoulement gazeux (258, 259) en forme annulaire, la section de diffusion (241) étant disposée au centre de la pluralité de chemins d'écoulement gazeux,
et
la pluralité de chemins d'écoulement gazeux (258, 259) ayant des diamètres différents les uns des autres afin de séparer des écoulements de gaz les uns des autres, et
la section de diffusion (241) ayant une saillie de diffusion (242) faisant saillie, sur une face arrière de la section de diffusion (241) faisant face à l'orifice d'entrée de gaz (215), en direction de l'orifice d'entrée de gaz (215),
**caractérisé en ce qu'**une hauteur de saillie de la saillie de diffusion (242) augmente vers un centre de la saillie de diffusion (242).

10. Sèche-cheveux selon la revendication 9, dans lequel la section de sortie de gaz (150) comprend :
une section centrale (154) afin d'émettre le gaz par celle-ci ; et
une section latérale (156) en forme annulaire entourant la section centrale (154), le gaz étant émis par la section latérale (156) et la section centrale (154),
la section de diffusion (241) étant disposée de manière à faire face à la section centrale et ayant un diamètre supérieur à un diamètre de la section centrale (156) afin de diffuser le gaz introduit par la section centrale (154).

11. Sèche-cheveux selon la revendication 10, dans lequel un diamètre de l'orifice d'entrée de gaz (215) est égal ou supérieur à un diamètre de la section latérale (156), de sorte que le gaz émis par la section centrale (154) et la section latérale (156) s'écoule dans le boîtier de diffuseur (210).

12. Sèche-cheveux selon la revendication 11, dans lequel le châssis de guidage (240) comprend en outre :
un premier guide (246) formé en forme annulaire de sorte que la section de diffusion (241) soit disposée au centre du premier guide (246), un premier chemin d'écoulement (258) pour l'écoulement du gaz étant défini entre le premier guide (246) et la section de diffusion (241) ; et
un second guide (251) formé en forme annulaire de sorte que la section de diffusion (241) et le premier guide (246) soient disposés au centre du second guide (251), un second chemin d'écoulement (259) pour l'écoulement du gaz étant défini entre le second guide (251) et la section de diffusion (241),
la section de diffusion (241) ayant un diamètre inférieur à un diamètre intérieur de la section latérale (156), et
la section latérale (156) étant positionnée de manière à faire face, dans une direction vers l'avant, à au moins une partie du premier chemin d'écoulement (258).

13. Sèche-cheveux selon la revendication 12, dans lequel le second chemin d'écoulement (259) a un diamètre intérieur supérieur à un diamètre extérieur de la section latérale (156), de sorte que le gaz introduit par la section centrale (154) et la section latérale (156) est diffusé et s'écoule.
